# EUROPEAN PATENT APPLICATION

(11) **EP 4 438 731 A1**
(43) Date of publication of application: **02.10.2024**
(21) Application number: 22897913.4
(22) Date of filing: 25.11.2022
(51) Int. Cl.: C12N 15/63, A61P 35/00, C12N 15/62, A61K 39/395, C07K 19/00, C07K 16/30, C12N 15/13

(54) **FUSION PROTEIN COMPRISING SIRP? MUTANT**

(30) Priority: 26.11.2021 CN 202111425620
(71) Applicant: Hangzhou Sumgen Biotech Co., Ltd., Hangzhou, Zhejiang 310056 (CN); Sumgen Mab (Beijing) Biotech Co., Ltd., Beijing 100176 (CN)
(72) Inventor: LV, Ming, Beijing 100176 (CN); DING, Xiaoran, Beijing 100176 (CN); MIAO, Shiwei, Beijing 100176 (CN); TAN, Bin, Beijing 100176 (CN)
(74) Representative: Weickmann & Weickmann PartmbB
(86) International application number: PCT/CN2022/134238
(87) International publication number: WO 2023/093831

(57) **Abstract**

The present application relates to a bifunctional fusion protein, which includes a first binding domain that specifically binds to CLDN18.2, and a second binding domain that specifically binds to a CD47 protein, wherein the second binding domain has at least one of the following characteristics: (a) binding to a CD47 protein at 4×10⁻⁸M or a lower *K_{D}* value; (b) specifically blocking the interaction between CD47 protein and S1RPα; (c) not causing a coagulation reaction; and (d) inhibiting the growth and/or proliferation of a tumor or tumor cells.

## Description

### TECHNICAL FIELD

The present application relates to the field of biomedicine, specifically to a multifunctional fusion protein that binds to CD47 and CLDN18.2, and use thereof in treating diseases and/or disorders.

### BACKGROUND

CD47 protein is a transmembrane glycoprotein and a member of the immunoglobulin superfamily. In addition to normal tissue cells expressing CD47, many tumor cells overexpress CD47. The binding of CD47 on the surface of tumor cells to SIRPα on the surface of macrophages prevents phagocytosis of tumor cells by macrophages, which is regarded as a mechanism for tumors to evade immune surveillance by the body. Blocking the interaction between CD47 protein and SIRPα can inhibit tumor growth.

Claudins are a family of tight junction membrane proteins that are expressed in epithelium and endothelium and form paracellular barriers and micropores that determine tight junction permeability. Claudin 18 isotype 2 (CLDN18.2), a splice variant of claudin 18, is a gastric antigen expressed on short-lived differentiated gastric epithelial cells. Deregulated expression of claudins can be detected in many cancers and may contribute to tumorigenesis and cancer invasion. The expression of CLDN18.2 increases in pancreatic ductal adenocarcinoma, esophageal tumors, non-small cell lung cancer, ovarian cancer, bile duct adenocarcinoma and cholangiocarcinoma.

### SUMMARY OF THE INVENTION

The present application provides a bifunctional fusion protein, which includes a portion that specifically binds to CLDN18.2 and a portion that specifically binds to CD47 protein. The fusion protein according to the present application can effectively inhibit the growth and/or proliferation of tumors or tumor cells.

In an aspect, the present application provides a bifunctional fusion protein comprising: a first binding domain that specifically binds to CLDN18.2; and a second binding domain that specifically binds to CD47 protein; wherein the second binding domain has at least one of the following properties: (a) binding to CD47 protein with a *K_{D}* value of 4×10⁻⁸M or lower; (b) specifically blocking the interaction between CD47 protein and SIRPα; (c) not inducing coagulation; and (d) inhibiting the growth and/or proliferation of tumors or tumor cells.

In some embodiments, the second binding domain comprises a mutant of human SIRPα variant 1, and the mutant comprises an amino acid mutation at one or more positions compared with the 33rd-149th amino acid sequence of SEQ ID NO: 22.

In some embodiments, the mutant in the fusion protein comprises an amino acid mutation at one or more amino acid positions selected from the group consisting of: L44, 161, V63, E77, Q82, K83, E84, V93, D95, L96, K98, N100, R107, G109 and V132.

In some embodiments, the mutant in the fusion protein comprises an amino acid substitution at amino acid residues 161 and E77.

In some embodiments, the mutant in the fusion protein comprises an amino acid substitution at amino acid residues 161 and V132.

In some embodiments, the mutant in the fusion protein comprises an amino acid substitution at amino acid residues 161 and E84.

In some embodiments, the mutant in the fusion protein comprises an amino acid substitution at amino acid residues 161, K83 and E84.

In some embodiments, the mutant in the fusion protein comprises an amino acid substitution at amino acid residues 161, G109 and V132.

In some embodiments, the mutant in the fusion protein comprises an amino acid substitution at amino acid residues 161, D95, L96, G109 and V132.

In some embodiments, the mutant in the fusion protein comprises an amino acid substitution at amino acid residues V93 and R107.

In some embodiments, the mutant in the fusion protein comprises an amino acid substitution at amino acid residues L44 and I61.

In some embodiments, the mutant in the fusion protein comprises an amino acid substitution at amino acid residues L44 and E77.

In some embodiments, the mutant in the fusion protein comprises an amino acid substitution at amino acid residues Q82, K83 and E84.

In some embodiments, the mutant in the fusion protein comprises an amino acid substitution at amino acid residues E77 and V132.

In some embodiments, the mutant in the fusion protein comprises an amino acid mutation at amino acid residues selected from the group consisting of:
(1) I61, V63, E77, E84, V93, L96, K98, N100 and V132;
(2) 161, E77, Q82, K83 and E84;
(3) 161, V63, K83, E84 and V132;
(4) 161, E77, E84, R107 and V132;
(5) 161, V63, E77, K83, E84 and N100;
(6) I61, E77, Q82, K83, E84 and R107;
(7) I61, E77, Q82, E84, V93, L96, N100, R107, G109 and V132;
(8) I61, E77, Q82, K83, E84 and V132;
(9) 161;
(10) I61, D95, L96, G109 and V132;
(11) 161, D95, L96, K98, G109 and V132;
(12) I61, E77, E84, V93, R107 and V132;
(13) E77, L96, N100, G109 and V132;
(14) I61, V63, Q82, E84, D95, L96, N100 and V132;
(15) 161, E77, Q82, K83, E84, V93, D95, L96, K98, N100 and V132;
(16) I61, E77, Q82, K83, E84 and V93;
(17) 161, V63, E77, K83, E84, D95, L96, K98 and N100;
(18) I61, V63, E77, K83, D95, L96, K98, N100 and G109;
(19) 161, E77, Q82, E84, V93, D95, L96, K98 and N100;
(20) 161, V63, E77, Q82 and E84; and
(21) L44, 161, E77, Q82, K83, E84 and V132.

In some embodiments, the mutant in the fusion protein comprises one or more amino acid substitutions selected from the group consisting of: L44V, I61L/V/F, V63I, E77I/N/Q/K/H/M/R/V/L, Q82S/R/G/N, K83R, E84Q/K/H/D/R/G, V93L/A, D95H/R/E, L96S/T, K98R, N100G/K/D/E, R107N/S, G109R/H and V132L/R/I/S.

In some embodiments, the mutant in the fusion protein comprises amino acid substitutions of I61L/V/F and E77Q/N/I.

In some embodiments, the mutant in the fusion protein comprises amino acid substitutions of I61L/V/F and V132I/S.

In some embodiments, the mutant in the fusion protein comprises amino acid substitutions of I61L/V/F, K83R and E84Q/D/H.

In some embodiments, the mutant in the fusion protein comprises amino acid substitutions of I61L/V/F, G109H and V132I/S.

In some embodiments, the mutant in the fusion protein comprises amino acid substitutions of I61L/V/F, D95H, L96S, G109H and V132I/S.

In some embodiments, the mutant in the fusion protein comprises amino acid substitutions of V93A and R107N.

In some embodiments, the mutant in the fusion protein comprises amino acid substitutions of L44V and I61L/V/F.

In some embodiments, the mutant in the fusion protein comprises amino acid substitutions of L44V and E77Q/N/I.

In some embodiments, the mutant in the fusion protein comprises amino acid substitutions of Q82S/R/G/N, K83R and E84Q/K/H/D/R/G.

In some embodiments, the mutant in the fusion protein comprises amino acid substitutions of E77Q/N/I and V132I/S.

In some embodiments, the mutant in the fusion protein comprises an amino acid mutation selected from the group consisting of:
(1) I61L, V63I, E77I, E84K, V93L, L96S, K98R, N100G and V132L;
(2) I61V, E77N, Q82S, K83R and E84H;
(3) I61F, V63I, K83R, E84K and V132I;
(4) I61L, E77Q, E84D, R107N and V132I;
(5) I61L, V63I, E77K, K83R, E84D and N100G;
(6) I61V, E77H, Q82R, K83R, E84H and R107S;
(7) I61L, E77I, Q82G, E84R, V93L, L96T, N100G, R107S, G109R and V132R;
(8) I61L, E77M, Q82G, K83R, E84D and V132L;
(9) I61L;
(10) I61F, D95H, L96S, G109H and V132S;
(11) I61F, D95H, L96S, K98R, G109H and V132S;
(12) I61L, E77Q, E84D, V93A, R107N and V132I;
(13) E77K, L96S, N100K, G109H and V132L;
(14) I61L, V63I, Q82G, E84G, D95R, L96S, N100D and V132I;
(15) I61L, E77R, Q82N, K83R, E84G, V93L, D95E, L96T, K98R, N100D and V132L;
(16) 161V, E77N, Q82S, K83R, E84H and V93A;
(17) I61V, V63I, E77V, K83R, E84D, D95E, L96T, K98R and N100E;
(18) I61L, V63I, E77V, K83R, D95E, L96S, K98R, N100D and G109R;
(19) I61V, E77L, Q82G, E84G, V93L, D95E, L96T, K98R and N100G;
(20) I61L, V63I, E77N, Q82G and E84G; and
(21) L44V, I61F, E77I, Q82R, K83R, E84Q and V132I.

In some embodiments, the mutant in the fusion protein further comprises one or more glycosylation site modifications.

In some embodiments, the glycosylation site modification comprises glycosylation site mutation.

In some embodiments, the glycosylation site mutation is located at position N110.

In some embodiments, the glycosylation mutation comprises an amino acid substitution of N110A.

In some embodiments, the mutant in the fusion protein comprises the amino acid sequences set forth in any one of SEQ ID NOs: 1-21.

In some embodiments, the first binding domain of the fusion protein comprises an antigen-binding protein targeting CLDN18.2.

In some embodiments, the antigen-binding protein comprises an antibody or an antigen-binding fragment thereof.

In some embodiments, the antibody is selected from the group consisting of monoclonal antibodies, single chain antibodies, chimeric antibodies, humanized antibodies, and fully human antibodies.

In some embodiments, the antigen-binding fragment is selected from the group consisting of Fab, Fab', F(ab)2, dAb, isolated complementarity determining region CDR, Fv and scFv.

In some embodiments, the CLDN18.2 comprises human CLDN18.2.

In some embodiments, the first binding domain comprises at least one CDR in a heavy chain variable region (VH), and the VH comprises the amino acid sequences set forth in SEQ ID NO: 24.

In some embodiments, the first binding domain comprises HCDR3, and the HCDR3 comprises the amino acid sequences set forth in SEQ ID NO: 25.

In some embodiments, the first binding domain comprises HCDR2, and the HCDR2 comprises the amino acid sequences set forth in SEQ ID NO: 26.

In some embodiments, the first binding domain comprises HCDR1, and the HCDR1 comprises the amino acid sequences set forth in SEQ ID NO: 27.

In some embodiments, the first binding domain comprises VH, and the VH comprises the amino acid sequences set forth in SEQ ID NO: 24.

In some embodiments, the first binding domain comprises an immunoglobulin single variable domain.

In some embodiments, the immunoglobulin single variable domain comprises V_{H}H.

In some embodiments, the first binding domain in the fusion protein comprises at least one CDR in V_{H}H, and the V_{H}H comprises the amino acid sequences set forth in SEQ ID NO: 24.

In some embodiments, the V_{H}H comprises HCDR3, and the HCDR3 comprises the amino acid sequences set forth in SEQ ID NO: 25.

In some embodiments, the V_{H}H comprises HCDR2, and the HCDR2 comprises the amino acid sequences set forth in SEQ ID NO: 26.

In some embodiments, the V_{H}H comprises HCDR1, and the HCDR1 comprises the amino acid sequences set forth in SEQ ID NO: 27.

In some embodiments, the V_{H}H comprises the amino acid sequences set forth in SEQ ID NO: 24.

In some embodiments, the first binding domain comprises a heavy chain constant region Fc fragment.

In some embodiments, the heavy chain constant region is derived from IgG.

In some embodiments, the heavy chain constant region is derived from human IgG.

In some embodiments, the IgG is selected from the group consisting of IgG1 and IgG4.

In some embodiments, the heavy chain constant region Fc fragment comprises the amino acid sequence of SEQ ID NO: 28.

In some embodiments, the first binding domain of the fusion protein is located at the N-terminus of the second binding domain.

In some embodiments, the fusion protein further includes a linker located at the C-terminus of the first binding domain and at the N-terminus of the second binding domain.

In some embodiments, the linker comprises the amino acid sequences set forth in SEQ ID NO: 24.

In some embodiments, the fusion protein comprises at least 2 second binding domains.

In some embodiments, each of the second binding domains of the fusion protein is located at the C-terminus of the first binding domain, respectively.

In some embodiments, the fusion protein comprises at least one polypeptide chain, wherein the polypeptide chain comprises the amino acid sequences set forth in any one of SEQ ID NOs: 30-35.

In some embodiments, the fusion protein comprises two polypeptide chains, and each polypeptide chain comprises the amino acid sequences set forth in any one of SEQ ID NOs: 30-35.

In another aspect, the present application further provides an immunoconjugate comprising the fusion protein.

In another aspect, the present application further provides one or more nucleic acid molecules encoding the fusion protein or the immunoconjugate.

In another aspect, the present application further provides a vector comprising the nucleic acid molecule.

In another aspect, the present application further provides a pharmaceutical composition comprising the fusion protein, the immunoconjugate, the nucleic acid molecule, and optionally a pharmaceutically acceptable carrier.

In another aspect, the present application further provides cells comprising the fusion protein, the immunoconjugate, the nucleic acid molecule or the vector.

In another aspect, the present application further provides a method for preparing the fusion protein, which includes culturing the cells under conditions enabling the expression of the fusion protein.

In another aspect, the present application further provides use of the fusion protein, the immunoconjugate, the nucleic acid molecule, the vector, the pharmaceutical composition, and the cell in the preparation of drugs for the prevention and/or treatment of diseases and/or disorders.

In some embodiments, the disease and/or disorder includes diseases and/or disorders associated with CLDN18.2.

In some embodiments, the diseases and/or disorders associated with CLDN18.2 include diseases involving cells expressing CLDN18.2 or diseases associated with cells expressing CLDN18.2.

In some embodiments, the disease and/or disorder includes tumors.

In some embodiments, the tumors include solid tumors and/or non-solid tumors.

In some embodiments, the tumors include gastric cancer, esophageal cancer, pancreatic cancer, lung cancer, ovarian cancer, colon cancer, liver cancer, head and neck cancer, and/or gallbladder cancer.

Those skilled in the art will readily appreciate other aspects and advantages of the present application from the detailed description below. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. A brief description of the drawings is as follows:
Fig. 1 shows an example of the structure of the fusion protein according to the present application.
Fig. 2 shows the results of binding of the fusion protein according to the present application to CD47.
Figs. 3A-3B show the results of binding of the fusion protein according to the present application to Raji cells.
Fig. 4 shows the results of binding of the fusion protein according to the present application to 293T/CLDN18.2 cells.
Fig. 5 shows the results of blocking CD47/SIRPα interaction by the fusion protein according to the present application.
Fig. 6 shows the detection of the ADCC activity of the fusion protein according to the present application.
Figs. 7A-7B show the detection of the binding activity of the fusion protein according to the present application to red blood cells.
Fig. 8 shows the growth curve of the mouse 293T/CLDN18.2 cell xenograft model after the start of treatment.

### DETAILED DESCRIPTION

The embodiments of the invention of the present application will be described below with specific examples. One skilled in the art can easily understand other advantages and effects of the invention of the present application from the disclosure of the specification.

### DEFINITION OF TERMS

In the present application, the term "fusion protein" generally refers to a polypeptide or protein consisting of two or more linked portions. Each of these portions can be a polypeptide or protein with different properties. The property may be a biological property, such as in vitro or in vivo activity. The property can also be a simple chemical or physical property, such as binding to a target molecule, catalysis of a reaction. The two portions can be linked directly via a single peptide bond or via a peptide linker. Fusion proteins can be artificially prepared through recombinant DNA technology. For example, genes or nucleic acid molecules encoding the two or more proteins or polypeptides can be linked to each other to form a fusion gene or fused nucleic acid molecule, and the fusion gene or fused nucleic acid molecule can encode the fusion protein. Translation of the fusion gene can produce a single polypeptide, which can have the properties of at least one, or even each, of the two or more proteins or polypeptides before fusion.

In the present application, the term "specific binding" or "specific" generally refers to measurable and reproducible interactions, such as binding between a target and an antibody, which may determine the presence of a target in the presence of a heterogeneous population of molecules, including biomolecules. For example, an antibody that specifically binds a target (which may be an epitope) may be an antibody that binds to that target with greater affinity, avidity, more readily, and/or for a greater duration than it binds to other targets. In some embodiments, the antibody specifically binds to an epitope on a protein, and the epitope is conserved among proteins in different species. In some embodiments, specific binding includes, but does not require, exclusive binding.

In the present application, the term "binding domain" is typically a domain that can specifically bind to and/or recognize a particular epitope on a target (e.g., antigen). In the present application, the term "domain" generally refers to substantially separate closely spaced spherical regions in a protein subunit structure. For example, a polypeptide chain can first form a regular secondary structure with adjacent amino acid residues in certain regions, and then the adjacent secondary structure fragments can be assembled together to form a super-secondary structure. On this basis, the polypeptide chain can fold into an approximately spherical tertiary structure. For larger protein molecules or subunits, in the polypeptide chains, two or more spatially distinct, relatively independent regional structures can be associated form a tertiary structure. This relatively independent regional structure can be called a domain.

In the present application, the terms "first" and "second" in the "first binding domain" and "second binding domain" may be used solely for differentiation in description.

In the present application, the term "CD47 protein" generally refers to an integrin-associated protein (IAP), which is a multi-pass transmembrane receptor belonging to the immunoglobulin superfamily. For example, the CD47 protein can bind to membrane integrins and bind to its ligands thrombospondin-1 (TSP-1) and signal-regulatory protein alpha (S1RPα). CD47 protein is widely expressed on the cell membrane surface. In the present application, the CD47 protein may include any variant, isotype, and species homolog of human CD47. The amino acid sequence of human CD47 protein is listed in GenBank as CEJ95640.1. The CD47 protein can be naturally expressed by cells or expressed on cells transfected with the CD47 gene.

In the present application, the term "SIRPα" generally refers to a regulatory membrane glycoprotein from the SIRP family, which may act as a ligand for the CD47 protein. In the present application, the SIRPα may include human S1RPα.

In the present application, the term "human SIRPα domain" generally refers to human SIRPα, a fragment or functional variant thereof, which is in a wild-type, endogenous mature form. In humans, the SIRPα protein is found to have two main forms. The amino acid sequence of one form (variant 1 or type V1) is listed as NCBI RefSeq NP_542970.1 (residues 31-504 constitute the mature form). Another form (variant 2 or type V2) differs from the variant 1 or type V1 by 13 amino acids and its amino acid sequence is listed in GenBank as CAA71403.1. These two forms of SIRPα make up approximately 80% of the various types of SIRPα present in humans. In the present application, the term "antigen-binding protein" generally refers to a protein that can bind to an antigen and is detached from its naturally occurring state. The "isolated antigen-binding protein" may comprise an antigen-binding portion and, optionally, a framework or skeleton portion that allows the antigen-binding portion to adopt a conformation that promotes the binding of the antigen-binding portion to the antigen. The antigen-binding protein may comprise, for example, antibody-derived protein framework regions (FRs) or alternative protein framework regions or artificial framework regions with grafted CDRs or CDR derivatives. Such frameworks include, but are not limited to, antibody-derived framework regions with mutations introduced, for example, to stabilize the three-dimensional structure of the antigen-binding protein, as well as fully synthetic framework regions comprising, for example, biocompatible polymers.

In the present application, the term "CDR", also known as "complementarity determining region", generally refers to a region in an antibody variable domain, the sequence of which is highly variable and/or forms a structural definition ring. Typically, an antibody includes six CDRs; three in VH (HCDR1, HCDR2, HCDR3), and three in VL (LCDR1, LCDR2, LCDR3). In some embodiments, naturally occurring camelid antibodies consisting solely of heavy chains function normally and stably in the absence of light chains. See, e.g., Hamers-Casterman et al., Nature 363:446-448 (1993); Sheriff et al, Nature Struct. Biol. 3:733-736 (1996). Antibody CDRs can be determined through a variety of coding systems, such as CCG, Kabat, AbM, Chothia, IMGT, Kabat/Chothia in combination. These coding systems are known in that art and can be referred to, for example, http://www.bioinf.org.uk/abs/index.html#kabatnum.

In the present application, the terms "variable domain" and "variable region" can be used interchangeably, and generally refer to a part of a heavy chain and/or light chain of an antibody. Variable domains of heavy and light chains can be referred to as "V_{H}" and "V_{L}" (or "VH" and "VL"), respectively. These domains are generally the most variable parts of an antibody (relative to other antibodies of the same type) and comprise antigen-binding sites.

In the present application, the term "antibody" generally refers to an immunoglobulin or a fragment thereof or a derivative thereof, covering any polypeptide including an antigen-binding site, whether produced in vitro or in vivo. The term includes, but is not limited to, polyclonal, monoclonal, monospecific, multispecific, nonspecific, humanized, single-chain, chimeric, synthetic, recombinant, hybrid, mutated and transplanted antibodies. Unless otherwise modified by the term "complete", such as in "complete antibodies", for purposes of the present invention, the term "antibody" also includes antibody fragments, such as Fab, F(ab')₂, Fv, scFv, Fd, ab and other antibody fragments that maintain antigen-binding function (e.g., specifically bind to CLDN 18.2).

In the present application, the term "antigen-binding fragment" generally refers to one or more fragments with the ability to specifically bind to an antigen (e.g., CLDN18.2). In the present application, the antigen- binding fragment may include Fab, Fab', F(ab)₂, Fv fragment, F(ab')₂, scFv, di-scFv and/or dAb.

In the present application, the term "Fab" generally refers to antigen-binding fragments of antibodies. As described above, intact antibodies can be digested using papain. The antibody is digested by papain to produce two identical antigen-binding fragments, namely the "Fab" fragment, and a residual "Fc" fragment (i.e., Fc region, ibid.). The Fab fragment can be composed of a complete L chain and the variable region of a heavy chain and the first constant region (CH1) of the H chain (VH). In the present application, the term "Fab' fragment" generally refers to a monovalent antigen-binding fragment of a human monoclonal antibody. For example, the Fab' fragment can include all of the light chain, all of the heavy chain variable regions and all or part of the first and second constant regions of the heavy chain. For example, the Fab' fragment may also include part or all of the heavy chain. In the present application, the term "F(ab')2" generally refers to antibody fragments produced by digestion of intact antibodies by pepsin. The F(ab')2 fragment contains two Fab fragments held together by disulfide bonds and part of the hinge region. F(ab')2 fragments have bivalent antigen-binding activity and are capable of cross-linking antigens. In the present application, the term "Fv fragment" generally refers to a monovalent antigen-binding fragment of a human monoclonal antibody, including all or part of heavy chain variable regions and light chain variable regions, and lacking heavy chain constant regions and light chain constant regions. Heavy chain variable regions and light chain variable regions include, for example, CDRs. In the present application, the term "scFv" generally refers to a fusion protein comprising at least one antibody fragment comprising a light chain variable region and at least one antibody fragment comprising a heavy chain variable region, wherein the light chain and the heavy chain variable regions are adjacent (e.g., via a synthetic linker such as a short flexible polypeptide linker) and can be expressed in the form of a single chain polypeptide, and wherein the scFv retains the specificity of the intact antibody of its origin. Unless otherwise specified, as used in the present application, the scFv may have the VL and VH variable regions in any order (e.g., relative to the N-terminus and C-terminus of the polypeptide), and the scFv may include VL-linker-VH or may include VH-linker-VL. In the present application, the term "dAb" generally refers to an antigen-binding fragment having a VH domain and a VL domain, or having either a VH domain or a VL domain, with reference to, for example, Ward et al. (Nature, 1989 Oct 12; 341 (6242): 544-6), with reference to Holt et al., Trends Biotechnol., 2003, 21(11): 484-490; and referring to other published patent applications such as WO 06/030220, WO 06/003388, and Domantis Ltd. The term "dAb" generally includes sdAb. The term "sdAb" generally refers to single domain antibodies. Single-domain antibodies usually refer to antibody fragments consisting only of an antibody heavy chain variable region (VH domain) or an antibody light chain variable region (VL). In some cases, single-domain antibodies can also comprise heavy chain constant regions.

In the present application, the term "monoclonal antibody" generally refers to antibody molecule preparations consisting of single molecules. Monoclonal antibodies are usually highly specific for a single antigenic site. Furthermore, unlike conventional polyclonal antibody preparations, which often have different antibodies directed against different determinants, each monoclonal antibody is directed against a single determinant on the antigen. In addition to their specificity, the advantage of monoclonal antibodies is that they can be synthesized by hybridoma cultures without contamination by other immunoglobulins. The modifier "monoclonal" represents the characteristics of antibodies obtained from a substantially homogeneous antibody population and is not interpreted as requiring the production of antibodies by any particular method. For example, the monoclonal antibodies used herein can be produced in hybridoma cells or can be produced by recombinant DNA methods.

In the present application, the term "chimeric antibody" generally refers to antibodies in which the variable region originates from one species and the constant region originates from another. In general, the variable region originates from antibodies of experimental animal such as rodents ("parent antibody"), and the constant region originates from human antibodies such that the resulting chimeric antibody is less likely to elicit an adverse immune response in a human individual than the parent antibody.

In the present application, the term "humanize antibody" generally refers to an antibody in which part or all of the amino acids other than the CDR region of a non-human antibody are replaced with corresponding amino acids derived from human immunoglobulin. Small additions, deletions, insertions, substitutions or modifications of amino acids in the CDR regions may also be allowed as long as they retain the ability of the antibody to bind to a specific antigen. The humanized antibody may optionally comprise at least a part of the human immunoglobulin constant region. "Humanized antibodies" retain antigen specificity similar to that of the original antibody. The "humanized" form of the non-human antibody may comprise, to a minimum, chimeric antibodies derived from sequences of the non-human immunoglobulin. In some cases, CDR region residues in human immunoglobulin (receptor antibody) can be replaced with CDR region residues of a non-human species (donor antibody) (such as alpaca, mouse, rat, rabbit or non-human primate) having the desired properties, affinity, and/or ability. In some cases, FR region residues of human immunoglobulin can be replaced with corresponding non-human residues. Furthermore, humanized antibodies may comprise amino acid modifications that are not found in the receptor antibody or in the donor antibody. These modifications may be made to further improve the performance of the antibody, such as binding affinity.

The proteins, polypeptides and/or amino acid sequences involved in the present application should also be understood to include at least the following range: variants or homologs that have the same or similar functions as the protein or polypeptide.

In the present application, the variant may be a protein or polypeptide (e.g., an antibody specifically binding to CLDN18.2 or a fragment thereof) with substitution, deletion or addition of one or more amino acids in the amino acid sequence of the protein and/or polypeptide. For example, the functional variant may comprise proteins or polypeptides with amino acid alterations through substitution, deletion and/or insertion of at least 1, such as 1-30, 1-20 or 1-10, such as 1, 2, 3, 4 or 5 amino acids. The functional variant may substantially retain the biological properties of the protein or polypeptide prior to the alteration (e.g., substitution, deletion, or addition). For example, the functional variant may retain at least 60%, 70%, 80%, 90%, or 100% of the biological activity (e.g., antigen-binding ability) of the protein or polypeptide prior to the alteration. For example, the substitutions may be conservative substitutions.

In the present application, the homolog may be a protein or polypeptide that shares at least about 85% (e.g., at least about 85%, about 90%, about 91%, about 92%, about 93%, about 94%, about 95%, about 96%, about 97%, about 98%, about 99% or higher) sequence homology with the amino acid sequence of the protein and/or the polypeptide (e.g. the antibody specifically binding to CLDN18.2 or fragment thereof).

In the present application, the homology generally refers to similarity, likeness or association between two or more sequences. "Percent sequence homology" can be calculated by comparing two sequences to be aligned in a comparison window to determine the number of positions with identical nucleic acid bases (e.g., A, T, C, G, I) or identical amino acid residues (e.g., Ala, Pro, Ser, Thr, Gly, Val, Leu, Ile, Phe, Tyr, Trp, Lys, Arg, His, Asp, Glu, Asn, Gln, Cys, and Met) in the two sequences to obtain the number of matching positions, dividing the number of matching positions by the total number of positions in the comparison window (i.e., window size), and multiplying the result by 100 to generate the percent sequence homology. Alignment for the purpose of determining percent sequence homology can be accomplished in a variety of ways known in the art, for example, using publicly available computer software such as BLAST, BLAST-2, ALIGN or Megalign (DNASTAR) software. One skilled in the art can determine appropriate parameters for aligning sequences, including any algorithms required to achieve maximal alignment within the full-length sequences being compared or within the sequence region of interest. The homology can also be determined by the following methods: FASTA and BLAST. A description of the FASTA algorithm can be found in W. R. Pearson and D. J. Lipman, "Improved Tool for Biological Sequence Comparison", (Proc. Natl. Acad. Sci. ), 85: 2444-2448, 1988; and D. J. Lipman and W. R. Pearson, "Fast and Sensitive Protein Similarity Search", Science, 227: 1435-1441, 1989. A description of the BLAST algorithm can be found in S. Altschul, W. Gish, W. Miller, E. W. Myers and D. Lipman, "A Basic Local Alignment Search Tool", Molecular Biology, 215: 403-410, 1990.

In the present application, the term "CLDN18.2", or "Claudin18.2", may be used interchangeably, typically referring to subtype 2 of the cell tight junction protein Claudin18. The term covers "full-length", unprocessed CLDN18.2, and any form of CLDN18.2 produced by cell processing. CLDN18.2 may include complete CLDN18.2 and fragments thereof, functional variants, isoforms, species homologs, derivatives, analogs thereof and analogs having at least one epitope in common with CLDN18.2.

Normally, in a polypeptide chain, the amino group is linked to another carboxyl group in the polypeptide chain to make it a chain, but at the two ends of the protein, there are remaining amino acid residues without peptide bonds, which are the end of the polypeptide chain end carrying a free amino group and the end of the polypeptide chain carrying a carboxyl group, respectively. In the present application, the term "N-terminus" generally refers to the end of a polypeptide chain in which the amino acid residue carry a free amino group. In the present application, the term "C-terminus" generally refers to the end of a polypeptide chain in which the amino acid residue carry a free carboxyl group.

In the present application, the term "nucleic acid molecule" generally refers to isolated forms of nucleotide, deoxyribonucleotides or ribonucleotides or analogs thereof of any length that are isolated from their natural environment or artificially synthesized.

In the present application, the term "immunoconjugate" generally refers to an immunocompetent polypeptide molecule conjugated with one or more heterologous molecules, including but not limited to cytotoxins. In the present application, "conjugate" and "link", "fuse" may be used interchangeably in the present application, and generally refer to the joining of two or more chemical elements, sequence or components together, for example by means including chemical conjugation or recombination. The heterologous molecule can be a cytotoxin, a chemotherapeutic drug, etc. For example, the immunoconjugate can be obtained by conjugating the fusion protein according to the present application to one or more heterologous molecules (e.g., cytotoxins).

In the present invention, the term "vector" refers to a nucleic acid delivery vehicle into which a polynucleotide encoding a protein can be inserted and its protein can be expressed. The host cells can be transformed, transduced or transfected with the vector so that the genetic elements it carries are expressed in the host cells. For example, vectors include: plasmids; phagemids; cosmids; artificial chromosomes such as yeast artificial chromosomes (YACs), bacterial artificial chromosomes (BACs) or P1-derived artificial chromosomes (PACs); bacteriophages such as λ phage or M13 phage and animal viruses. The types of animal viruses used as vectors include retroviruses (including lentiviruses), adenoviruses, adeno-associated viruses, herpes viruses (such as herpes simplex virus), poxviruses, baculoviruses, papillomaviruses, and papovaviruses (such as SV40). A vector may contain a variety of elements that control expression, including promoter sequences, transcription initiation sequences, enhancer sequences, selection elements, and reporter genes. In addition, the vector may also contain a replication initiation site. Vectors may also contain components that facilitate its entry into cells, such as viral particles, liposomes, or protein coats, but are not limited thereto.

In the present application, the term "pharmaceutical composition" generally refers to a composition for use in the prevention/treatment of a disease or condition. The pharmaceutical composition may comprise the isolated antigen-binding protein according to the present application, the nucleic acid molecule according to the present application, the vector according to the present application, and/or the cell according to the present application, and optionally a pharmaceutically acceptable adjuvant. In addition, the pharmaceutical composition may further comprise suitable preparations of one or more (pharmaceutically effective) carriers, stabilizers, excipients, diluents, solubilizers, surfactants, emulsifiers and/or preservatives. Acceptable ingredients of the composition are preferably non-toxic to the recipient at the doses and concentrations used. Pharmaceutical compositions of the present invention include, but are not limited to, liquid, frozen and lyophilized compositions.

In the present application, the term "pharmaceutically acceptable carrier" generally includes pharmaceutically acceptable carriers, excipients or stabilizers at doses and concentrations that are non-toxic to the cells or mammals to which they are exposed.

In the present application, the term "tumor" generally refers to neoplasms in a mammalian organism (e.g., cells or components thereof) that results from the proliferation of local tissue cells under the action of various tumorigenic factors. In the present application, tumors may include solid tumors and non-solid tumors. In the present application, the tumors may include gastric cancer, esophageal cancer, pancreatic cancer, lung cancer, ovarian cancer, colon cancer, liver cancer, head and neck cancer, and/or gallbladder cancer.

For the purposes of the present application, the term "comprising" generally refers to the inclusion of clearly specified features, but does not exclude other elements.

In the present application, the term "approximately" generally refers to a change in the range of 0.5% to 10% above or below a specified value, for example, a change in the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### DETAILED DESCRIPTION OF THE INVENTION

### Fusion protein

In one aspect, the present application provides a fusion protein, which may comprise a first binding domain and a second binding domain. The first binding domain may specifically bind to CLDN18.2; the second binding domain may specifically bind to CD47 protein, wherein the second binding domain may have at least one of the following properties: (a) binding to CD47 protein with a *K_{D}* value of 4×10⁻⁸M or lower; (b) specifically blocking the interaction between CD47 protein and SIRPα; (c) not inducing coagulation; and (d) being capable of inhibiting the growth and/or proliferation of tumors or tumor cells.

In some embodiments, the second binding domain may comprise a mutant of SIRPα variant 1 or a fragment thereof. Compared with the 33rd to 149th amino acid sequence of SEQ ID NO: 22, it comprises an amino acid residue mutation at one or more (for example, 1-2, 1-3, 1-4, 1-5, 1-6, 1-7, 1-8, 1-9, 1-10 or more) positions. The amino acid residue mutation may include substitution, deletion or addition of amino acid residues. The position of the amino acid is determined by the amino acid position as set forth in SEQ ID NO: 22. For example, I61 usually refers to the 61st amino acid based on the amino acid sequences set forth in SEQ ID NO: 22. In the present application, the amino acid sequences set forth in SEQ ID NO: 23 may refer to the 33rd-149th amino acid residues of human SIRPα variant 1 (SEQ ID NO: 22), that is, the position of the first amino acid of the amino acid sequences set forth in SEQ ID NO: 23 is numbered as "position 33".

In the present application, the mutant of SIRPα variant 1 may include one or more amino acid mutations in the 33rd to 149th amino acid sequence of SIRPα variant 1. For example, the mutant of SIRPα variant 1 can comprise one or more amino acid mutations in the amino acid sequences set forth in SEQ ID NO: 23. For example, the mutant of SIRPα variant 1 according to the present application comprises one or more amino acid mutations compared with the amino acid sequences set forth in SEQ ID NO: 23.

In the present application, the fusion protein can simultaneously bind to tumor-associated antigens and CD47 protein, thereby playing a role in treating diseases and/or disorders.

In the present application, the term "first bin domain" typically refers to a domain that may specifically bind to CLDN18.2. The term "second binding domain" generally refers to domains that specifically bind to CD47 protein.

### The second binding domain that specifically binds to CD47 protein

In the present application, the mutant (e.g., a mutant of human SIRPα variant 1 that specifically binds to CD47 protein) comprises an amino acid substitution at one or more (e.g., 1-2, 1-3, 1-4, 1-5, 1-6, 1-7, 1-8, 1-9, 1-10 or more) amino acid residues selected from the group consisting of: L44, I61 , V63, E77, Q82, K83, E84, V93, D95, L96, K98, N100, R107, G109 and V132.

For example, the mutant may comprise an amino acid mutation at position L44. For example, the mutant may comprise an amino acid mutation at position I61. For example, the mutant may comprise an amino acid mutation at position V63. For example, the mutant may comprise an amino acid mutation at position E77. For example, the mutant may comprise an amino acid mutation at position Q82. For example, the mutant may comprise an amino acid mutation at position K83. For example, the mutant may comprise an amino acid mutation at position E84. For example, the mutant may comprise an amino acid mutation at position V93. For example, the mutant may comprise an amino acid mutation at position D95. For example, the mutant may comprise an amino acid mutation at position L96. For example, the mutant may comprise an amino acid mutation at position K98. For example, the mutant may comprise an amino acid mutation at position N100. For example, the mutant may comprise an amino acid mutation at position R107. For example, the mutant may comprise an amino acid mutation at position G109. For example, the mutant may comprise an amino acid mutation at position V132.

In the present application, the position of an amino acid residue in the amino acid substitution is the residue number determined based on the amino acid sequences set forth in SEQ ID NO: 22.

"Amino acid substitution Xn" means that amino acid substitution occurs at the residue X at the nth position corresponding to the amino acid sequences set forth in SEQ ID NO: 22, where n is a positive integer and X is an abbreviation for any amino acid residue. For example, "amino acid substitution I61" indicates that amino acid substitution occurs at residue I at position 61 corresponding to the amino acid sequences set forth in SEQ ID NO: 22.

In the present application, the amino acid substitution may be a non-conservative substitution. The non-conservative substitution may include changing the amino acid residues in the target protein or polypeptide in a non-conservative manner, for example, changing an amino acid residue with a certain side chain size or a certain property (for example, hydrophilicity) to an amino acid residue with a different side chain size or a different property (e.g., hydrophobicity).

In the present application, the amino acid substitution may also be a conservative substitution. The conservative substitution may include changing the amino acid residues in the target protein or polypeptide in a non-conservative manner, for example, changing an amino acid residue with a certain side chain size or a certain property (for example, hydrophilicity) to an amino acid residue with the same or similar side chain size or the same or similar property (e.g., still hydrophilicity). Such conservative substitutions generally do not have a significant impact on the structure or function of the resulting protein. In the present application, the amino acid sequence variant as the fusion protein or fragments thereof may include conservative amino acid substitutions that do not significantly change the structure of the protein or its function (for example, mutants that block CD47 and human SIRPα variant 1 that specifically binds to CD47 protein).

As an example, substitutions between amino acids within each of the following groups may be considered conservative substitutions in the present application: group of amino acids with nonpolar side chains: alanine, valine, leucine, isoleucine, proline, phenylalanine, tryptophan and methionine. Group of uncharged amino acids with polar side chains: glycine, serine, threonine, cysteine, tyrosine, asparagine, and glutamine. Group of negatively charged amino acids with polar side chains: aspartic acid and glutamic acid. Positively charged basic amino acids: lysine, arginine, and histidine. Amino acids with phenyl: phenylalanine, tryptophan, and tyrosine.

In the present application, the mutant may comprise amino acid substitutions at amino acid residues selected from the group consisting of: (1) I61, V63, E77, E84, V93, L96, K98, N100 and V132; (2) I61, E77, Q82, K83 and E84; (3) I61, V63, K83, E84 and V132; (4) I61, E77, E84, R107 and V132; (5) I61, V63, E77, K83, E84 and N100; ( 6) I61, E77, Q82, K83, E84 and R107; (7) I61, E77, Q82, E84, V93, L96, N100, R107, G109 and V132; (8) I61, E77, Q82, K83, E84 and V132; (9) I61; (10) I61, D95, L96, G109 and V132; (11) I61, D95, L96, K98, G109 and V132; (12) I61, E77, E84, V93, R107 and V132; (13) E77, L96, N100, G109 and V132; (14) I61, V63, Q82, E84, D95, L96, N100 and V132; (15) I61, E77, Q82, K83, E84, V93, D95, L96, K98, N100 and V132; (16) I61, E77, Q82, K83, E84 and V93; (17) I61, V63, E77, K83, E84, D95, L96, K98 and N100; (18) I61, V63, E77, K83, D95, L96, K98, N100 and G109; (19) I61, E77, Q82, E84, V93, D95, L96, K98 and N100; (20) I61, V63, E77, Q82 and E84; and (21) L44, I61, E77, Q82, K83, E84 and V132.

In the present application, the mutant may comprise one or more (for example, 1-2, 1-3, 1-4, 1-5, 1-6, 1-7, 1-8, 1-9, 1-10 or more) amino acid substitutions selected from the group consisting of: L44V, I61L/V/F, V63I, E77I/N/Q/K/H/M/R /V/L, Q82S/R/G/N, K83R, E84Q/K/H/D/R/G, V93L/A, D95H/R/E, L96S/T, K98R, N100G/K/D/E, R107N/S, G109R/H and V132L/R/I/S.

In the present application, the amino acid substitution "XnY/Z" means that the residue X at the nth position corresponding to the amino acid sequences set forth in SEQ ID NO: 22 is substituted with the amino acid residue Y or the amino acid residue Z, wherein n is a positive integer, and X, Y and Z are each independently an abbreviation of any amino acid residue, and X is different from Y or Z. For example, the amino acid substitution "I61L/V/F" means that the residue I at position 61 corresponding to the amino acid sequences set forth in SEQ ID NO: 22 is substituted with the amino acid residue L, V or F.

For example, the mutant may comprise L44V amino acid mutation. For example, the mutant may comprise I61L, I61V or I61F amino acid mutation. For example, the mutant may comprise V63I amino acid mutation. For example, the mutant may comprise E77I, E77N, E77Q, E77K, E77H, E77M, E77R, E77V or E77L amino acid mutation. For example, the mutant may comprise Q82S, Q82R, Q82G or Q82N amino acid mutation. For example, the mutant may comprise K83R amino acid mutation. For example, the mutant may comprise E84Q, E84K, E84H, E84D, E84R or E84G amino acid mutation. For example, the mutant may comprise V93L or V93A amino acid mutation. For example, the mutant may comprise D95H, D95R or D95E amino acid mutation. For example, the mutant may comprise L96S or L96T amino acid mutation. For example, the mutant may comprise K98R amino acid mutation. For example, the mutant may comprise N100G, N100K, N100D or N100E amino acid mutation. For example, the mutant may comprise R107N or R107S amino acid mutation. For example, the mutant may comprise G109R or G109H amino acid mutation. For example, the mutant may comprise V132L, V132R, V132I or V132S amino acid mutation.

In the present application, the mutants may comprise substitutions at the following amino acid positions: I61 and E77. For example, the mutant may comprise the amino acid substitutions of I61L/V/F and E77Q/N/I.

In the present application, the mutant may comprise substitutions at the following amino acid positions: I61 and V132. For example, the mutant may comprise the amino acid substitutions of I61L/V/F and V132I/S.

In the present application, the mutant may comprise substitutions at the following amino acid positions: I61 and E84. For example, the mutant may comprise the amino acid substitutions of I61L/V/F and E84D/H.

In the present application, the mutant may comprise substitutions at the following amino acid positions: I61, K83 and E84. For example, the mutant may comprise the amino acid substitutions of I61L/V/F, K83R and E84Q/D/H.

In the present application, the mutant may comprise substitutions at the following amino acid positions: I61 D95 and L96. For example, the mutant may comprise the amino acid substitutions of I61L/V/F, D95H and L96S.

In the present application, the mutant may comprise substitutions at the following amino acid positions: I61, D95, L96, G109 and V132. For example, the mutant may comprise the amino acid substitutions of I61L/V/F, D95H, L96S, G109H and V132I/S.

In the present application, the mutant may comprise substitutions at the following amino acid positions: I61, G109 and V132. For example, the mutant may comprise the amino acid substitutions of I61L/V/F, G109H and V 132I/S.

In the present application, the mutant may comprise substitutions at the following amino acid positions: V93 and R107. For example, the mutant may comprise the amino acid substitutions of V93A and R107N.

In the present application, the mutant may comprise substitutions at the following amino acid positions: L44 and I61. For example, the mutant may comprise the amino acid substitutions of L44V and I61L/V/F.

In the present application, the mutant may comprise substitutions at the following amino acid positions: L44 and E77. For example, the mutant may comprise the amino acid substitutions of L44V and E77Q/N/I.

In the present application, the mutant may comprise substitutions at the following amino acid positions: Q82, K83 and E84. For example, the mutant may comprise the amino acid substitutions of Q82S/R/G/N, K83R and E84Q/K/H/D/R/G.

In the present application, the mutant may comprise substitutions at the following amino acid positions: E77 and V132. For example, the mutant may comprise the amino acid substitutions of E77Q/N/I and V132I/S.

In the present application, the mutant may comprise amino acid substitutions selected from the group consisting of: (1) I61L, V63I, E77I, E84K, V93L, L96S, K98R, N100G and V132L; (2) I61V, E77N, Q82S, K83R and E84H; (3) I61F, V63I, K83R, E84K and V132I; (4) I61L, E77Q, E84D, R107N and V132I; (5) I61L, V63I, E77K, K83R, E84D and N100G; (6) I61V, E77H, Q82R, K83R, E84H and R107S; (7) I61L, E77I, Q82G, E84R, V93L, L96T, N100G, R107S, G109R and V132R; (8) I61L, E77M, Q82G, K83R, E84D and V132L; (9) I61L; (10) I61F, D95H, L96S, G109H and V132S; (11) I61F, D95H, L96S, K98R, G109H and V132S; (12) I61L, E77Q, E84D, V93A, R107N and V132I; (13) E77K, L96S, N100K, G109H and V132L; (14) I61L, V63I, Q82G, E84G, D95R, L96S, N100D and V132I; (15) I61L, E77R, Q82N, K83R, E84G, V93L, D95E, L96T, K98R, N100D and V132L; (16) 161V, E77N, Q82S, K83R, E84H and V93A; (17) 161V, V63I, E77V, K83R, E84D, D95E, L96T, K98R and N100E; (18) I61L, V63I, E77V, K83R, D95E, L96S, K98R, N100D and G109R; (19) 161V, E77L, Q82G, E84G, V93L, D95E, L96T, K98R and N100G; (20) I61L, V63I, E77N, Q82G and E84G; and (21) L44V, I61F, E77I, Q82R, K83R, E84Q and V132I.

In the present application, on the basis of the truncated domain of human SIRPα variant 1 (the amino acid sequences set forth in SEQ ID NO: 23, that is, 33rd-149th residues in the amino acid sequence of human SIRPα variant 1), the mutants of SIRPα variant 1 comprising the above amino acid substitution groups (1) - (21) respectively can be named M1, M5, M12, M35, M37, M41, M57, M67, M81, M82, M84, M91, M99, M102, M111, M122, M126, M130, M135, M145 and M169 in sequence.

In the present application, the mutant may also comprise one or more glycosylation site modifications. The modification involves changing the glycosylation site through artificial means. For example, the glycosylation site modification comprises glycosylation site mutation. In some embodiments, the glycosylation site mutation is located at position N110. In some embodiments, the glycosylation site mutation comprises N110A.

In the present application, compared with the amino acid sequences set forth in SEQ ID NO: 23, the mutant (for example, the mutant of human SIRPα variant 1 that specifically binds to CD47 protein) comprises amino acid substitutions at one or more amino acid residues selected from the group consisting of: L44, 161, V63, E77, Q82, K83, E84, V93, D95, L96, K98, N100, R107, G109, N110 and V132.

In the present application, the mutant in the fusion protein may comprise amino acid substitutions at amino acid residues selected from the group consisting of: (1) I61, V63, E77, E84, V93, L96, K98, N100, N110 and V132; (2) I61, E77, Q82, K83, E84 and N110; (3) I61, V63, K83, E84, N110 and V132; (4) 161, E77, E84, R107\N110 and V132; (5) 161, V63, E77, K83, E84, N100 and N110; (6) 161, E77, Q82, K83, E84, R107 and N110; (7) 161, E77, Q82, E84, V93, L96, N100, R107, G109, N110 and V132; (8) 161, E77, Q82, K83, E84, V132 and N110; (9) I61 and N110; (10) I61, D95, L96, G109, N110 and V132; (11) I61, D95, L96, K98, G109, N110 and V132; (12) I61, E77, E84, V93, R107, N110 and V132; (13) E77, L96, N100, G109, N110 and V132; (14) I61, V63, Q82, E84, D95, L96, N100, N110 and V132; (15) I61, E77, Q82, K83, E84, V93, D95, L96, K98, N100, N110 and V132; (16) I61, E77, Q82, K83, E84, V93 and N110; (17) 161, V63, E77, K83, E84, D95, L96, K98, N100 and N110; (18) 161, V63, E77, K83, D95, L96, K98, N100, G109 and N110; (19) 161, E77, Q82, E84, V93, D95, L96, K98, N100 and N110; (20) 161, V63, E77, Q82, E84 and N110; and (21) L44V, I61F, E77I, Q82R, K83R, E84Q, N110A and V132I.

In the present application, the mutant in the fusion protein may comprise amino acid substitutions selected from the group consisting of: (1) I61L, V63I, E77I, E84K, V93L, L96S, K98R, N100G, N110A and V132L; (2) I61V, E77N, Q82S, K83R, E84H and N110A; (3) I61F, V63I, K83R, E84K, N110A and V132I; (4) I61L, E77Q, E84D, R107N, N110A and V132I; (5) I61L, V63I, E77K, K83R, E84D, N100G and N110A; (6) I61V, E77H, Q82R, K83R, E84H, R107S and N110A; (7) I61L, E77I, Q82G, E84R, V93L, L96T, N100G, R107S, G109R, N110A and V132R; (8) I61L, E77M, Q82G, K83R, E84D, N110A and V132L; (9) I61L and N110A; (10) I61F, D95H, L96S, G109H, N110A and V132S; (11) I61F, D95H, L96S, K98R, G109H, N110A and V132S; (12) I61L, E77Q, E84D, V93A, R107N, N110A and V132I; (13) E77K, L96S, N100K, G109H, N110A and V132L; (14) I61L, V63I, Q82G, E84G, D95R, L96S, N100D, N110A and V132I; (15) I61L, E77R, Q82N, K83R, E84G, V93L, D95E, L96T, K98R, N100D, N110A and V132L; (16) 161V, E77N, Q82S, K83R, E84H, V93A and N110A; (17) 161V, V63I, E77V, K83R, E84D, D95E, L96T, K98R, N100E and N110A; (18) I61L, V63I, E77V, K83R, D95E, L96S, K98R, N100D, G109R and N110A; (19) 161V, E77L, Q82G, E84G, V93L, D95E, L96T, K98R, N100G and N110A; (20) I61L, V63I, E77N, Q82G, E84G and N110A; and (21) L44V, I61F, E77I, Q82R, K83R, E84Q, N110A and V132I.

In the present application, on the basis of the truncated domain of human SIRPα variant 1 (the amino acid sequences set forth in SEQ ID NO: 23, that is, 33rd-149th residues in the amino acid sequence of human SIRPα variant 1), the mutants of SIRPα variant 1 comprising the above amino acid substitution groups (1) - (21) respectively can be named M1N, M5N, M12N, M35N, M37N, M41N, M57N, M67N, M81N, M82N, M84N, M91N, M99N, M102N, M111N, M122N, M126N, M130N, M135N, M145N and M169N in sequence. The mutant of SIRPα variant 1 may comprise the amino acid sequences set forth in SEQ ID NOs: 1-21 in sequence. In the present application, the mutant of SIRPα variant 1 may comprise an amino acid sequence having at least 80% (e.g., at least 85%, at least 90%, at least 91%, at least 92%, at least 93%, at least 94%, at least 95%, at least 96%, at least 97%, at least 98%, at least 99%, or at least 100%) sequence homology with the amino acid sequences set forth in any one of SEQ ID NOs: 1-21.

### The first binding domain that specifically binds to CLDN18.2

In the present application, the first binding domain comprises an antigen-binding protein. In the present application, the antigen-binding protein may comprise an antibody or antigen-binding fragment. In some embodiments, the antibody is selected from the group consisting of monoclonal antibodies, single chain antibodies, chimeric antibodies, humanized antibodies, and fully human antibodies. In some embodiments, the antigen-binding fragment is selected from the group consisting of Fab, Fab', F(ab')2, F(ab)2, dAb, isolated complementarity determining region CDR, Fv and scFv.

In some embodiments, the antigen-binding protein is capable of binding to CLDN18.2.

In the present application, the antigen-binding protein may comprise at least one CDR of an antibody heavy chain variable region, and the antibody heavy chain variable region may comprise the amino acid sequences set forth in SEQ ID NO: 24. For example, the CDR can be classified according to the IMGT numbering method.

In some embodiments, the antigen-binding protein comprises HCDR3, and the HCDR3 comprises the amino acid sequences set forth in SEQ ID NO: 25.

In the present application, the antigen-binding protein can comprise HCDR2, and the HCDR2 can comprise the amino acid sequences set forth in SEQ ID NO: 26.

In the present application, the antigen-binding protein may comprise HCDR1, and the HCDR1 may comprise the amino acid sequences set forth in SEQ ID NO: 27.

In the present application, the antigen-binding protein may comprise HCDR1, HCDR2 and HCDR3. The HCDR1 may comprise the amino acid sequences set forth in SEQ ID NO: 27, the HCDR2 may comprise the amino acid sequences set forth in SEQ ID NO: 26, and the HCDR3 may comprise the amino acid sequences set forth in SEQ ID NO: 25.

In the present application, the antigen-binding protein may comprise VH, and the VH may comprise the amino acid sequences set forth in SEQ ID NO: 24.

In the present application, the antigen-binding protein may comprise an immunoglobulin single variable domain. In the present application, the antigen-binding protein may comprise V_{H}H. For example, the antigen-binding protein may comprise at least one CDR in V_{H}H, and the V_{H}H comprises the amino acid sequences set forth in SEQ ID NO: 24. For example, the CDR can be classified according to the IMGT numbering method.

In the present application, the V_{H}H can comprise HCDR3, and the HCDR3 can comprise the amino acid sequences set forth in SEQ ID NO: 25.

In the present application, the V_{H}H may comprise HCDR2, and the HCDR2 may comprise the amino acid sequences set forth in SEQ ID NO: 26.

In the present application, the V_{H}H may comprise HCDR1, and the HCDR1 may comprise the amino acid sequences set forth in SEQ ID NO: 27.

In the present application, the V_{H}H may comprise HCDR1, HCDR2 and HCDR3. The HCDR1 may comprise the amino acid sequences set forth in SEQ ID NO: 27, the HCDR2 may comprise the amino acid sequences set forth in SEQ ID NO: 26, and the HCDR3 may comprise the amino acid sequences set forth in SEQ ID NO: 25.

In the present application, the antigen-binding protein may comprise an Fc fragment of a heavy chain constant region of an antibody. In the present application, the heavy chain constant region of the antigen-binding protein may be derived from IgG. In the present application, the heavy chain constant region may be derived from human IgG. The IgG according to the present application may be selected from IgG1 and IgG4. In the present application, the Fc region of the antibody heavy chain constant region may comprise the amino acid sequences set forth in SEQ ID NO: 28.

### How the first binding domain and the second binding domain are linked

In the present application, the first binding domain may be located at the N-terminus of the second binding domain. For example, the C-terminus of the first binding domain may be directly or indirectly linked to the N-terminus of the second binding domain. For example, the C-terminus of the first binding domain can be indirectly linked to the N-terminus of the second binding domain through a linker. For example, the C-terminus of the first binding domain may also be directly linked to the N-terminus of the second binding domain.

In the present application, the fusion protein may further comprise a linker, and the linker may be located at the C-terminus of the first binding domain and at the N-terminus of the second binding domain. For example, in the fusion protein, the C-terminus of the first binding domain can be linked to the N-terminus of the linker, and the C-terminus of the linker can be linked to the N-terminus of the second binding domain. For example, the fusion protein may include the first binding domain, the linker and the second binding domain in sequence from the N-terminus to the C-terminus.

In the present application, the linker may comprise the amino acid sequences set forth in SEQ ID NO: 29.

In some cases, the fusion protein may comprise at least 2 (e.g., at least 2, at least 3, at least 4, at least 5, at least 6, at least 7, at least 8, at least 9, at least 10, or more) second binding domains. In the present application, each of the second binding domains may be located at the C-terminus of the first binding domain, respectively. In the present application, the two or more second binding domains may be directly or indirectly linked to the C-terminus of the first binding domain, respectively.

In the present application, the fusion protein may comprise a first binding domain that specifically binds to CLDN18.2, and a second binding domain that specifically binds to CD47 protein, wherein the second binding domain may comprise a mutant of human SIRPα variant 1, and the C-terminus of the antibody that specifically binds CLDN18.2 or an antigen-binding fragment or variant thereof can be directly or indirectly linked to the N-terminus of the mutant of human SIRPα variant 1. For example, the second binding domain may comprise at least two mutants of human SIRPα variant 1, and the N-terminus of each of the two mutants of human SIRPα variant 1 is linked to the C-terminus of an antibody that specifically binds to CLDN18.2 or an antigen-binding fragment or variant thereof, respectively.

In the present application, the fusion protein may comprise at least one polypeptide chain, wherein the polypeptide chain may sequentially comprise V_{H}H, an Fc domain, a linker and a mutant of human SIRPα variant 1 capable of specifically binding to CLDN18.2 from N-terminus to C-terminus. In the present application, the fusion protein may comprise two polypeptide chains, wherein each of the polypeptide chains may sequentially comprise V_{H}H, an Fc domain, a linker and a mutant of human SIRPα variant 1 capable of specifically binding to CLDN18.2 from N-terminus to C-terminus, respectively. For example, the fusion protein may comprise two polypeptide chains, and the two polypeptide chains may comprise the same amino acid sequence, for example, the amino acid sequences set forth in any one of SEQ ID NOs: 30-35.

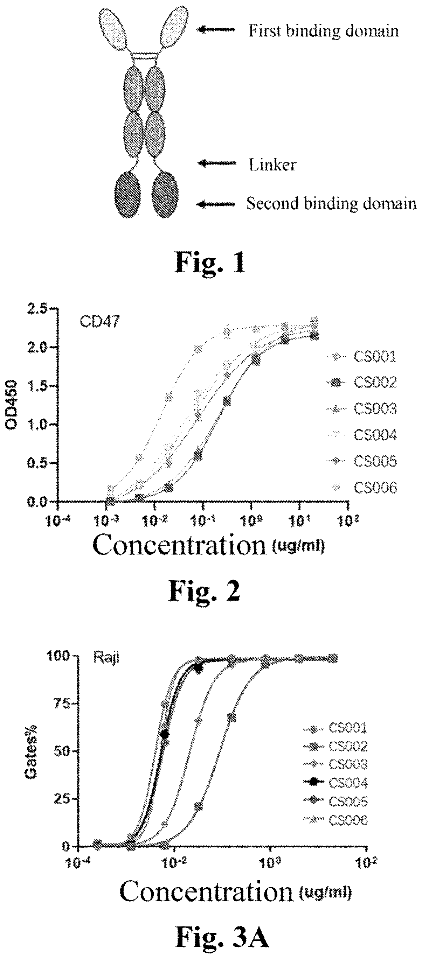

For example, as shown in Fig. 1, the first binding domain of the fusion protein may comprise NA3S-H1, and the second binding domain of the fusion protein may comprise a mutant of human SIRPα variant 1. The first binding domain and the second binding domain may be linked by a linker. The linker may comprise the amino acid sequences set forth in SEQ ID NO: 29.

For example, the polypeptide chain of the fusion protein may comprise the amino acid sequences set forth in SEQ ID NO: 24, SEQ ID NO: 28, SEQ ID NO: 29 and SEQ ID NO: 1 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 2 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 3 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 4 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 5 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 6 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 7 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 8 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 9 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 10 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 11 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 12 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 13 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 14 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 15 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 16 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 17 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 18 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 19 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 20 in sequence from the N terminus to the C terminus, respectively.

For example, the polypeptide chain of the fusion protein may comprise the amino acid sequences set forth in SEQ ID NO: 24, SEQ ID NO: 28, SEQ ID NO: 29 and SEQ ID NO: 21 in sequence from the N terminus to the C terminus, respectively.

### Immunoconjugates, nucleic acid molecules, vectors and cells and preparation methods

In another aspect, the present application further provides an immunoconjugate comprising the fusion protein. For example, the immunoconjugate can be a fusion protein-drug conjugate (ADC), wherein the fusion protein according to the present application is conjugated to one or more therapeutic agents, including but not limited to cytotoxic agents, radiotoxic agents (e.g., radioactive isotopes), and/or immunosuppressive agents (e.g., any agent that kills cells by inhibiting immune response), etc. In some embodiments, the therapeutic agent may be a therapeutic agent capable of treating a tumor-related disease or disorder.

The conjugation can be performed through a peptide linker (e.g., a cleavable linker) or other means. For example, the linker can be an acid-labile linker, a peptidase-sensitive linker, a light-labile linker, etc.

In another aspect, the present application provides one or more isolated nucleic acid molecules encoding the fusion protein or the immunoconjugate. For example, each of the one or more nucleic acid molecules can encode the complete antibody or antigen-binding fragment thereof, or can encode a portion thereof (e.g., one or more of HCDR1-3, LCDR1-3, VL, VH, light chain or heavy chain).

The nucleic acid molecules according to the present application may be isolated. For example, it may be produced or synthesized by: (i) amplification in vitro, such as amplification by polymerase chain reaction (PCR), (ii) clonal recombination, (iii) purification, such as by enzymatic digestion and gel electrophoresis fractionation, or (iv) synthesis, such as chemical synthesis. In some embodiments, the isolated nucleic acid is a nucleic acid molecule prepared by recombinant DNA technology.

Recombinant DNA and molecular cloning technologies include those described in Sambrook, J., Fritsch, E. F., and Maniatis, T. Molecular Cloning: A Laboratory Manual; Cold Spring Harbor Laboratory Press: Cold Spring Harbor, (1989) (Maniatis) and T. J. Silhavy, M. L. Bennan and L. W. Enquist, Experiments with Gene Fusions, Cold Spring Harbor Laboratory, Cold Spring Harbor, N.Y. (1984), and Ausubel, F. M. et al., Current Protocols in Molecular Biology, pub. by Greene Publishing Assoc. and Wiley-Interscience (1987). Briefly, the nucleic acids can be prepared from genomic DNA fragments, cDNA and RNA. All of the nucleic acids can be extracted directly from cells or recombinantly produced by various amplification methods including, but not limited to, PCR and RT-PCR.

Direct chemical synthesis of nucleic acids typically involves the sequential addition of 3'-blocked and 5'-blocked nucleotide monomers to the terminal 5'-hydroxyl group of a growing nucleotide polymer chain, where each addition is achieved through nucleophilic attack on the terminal 5'-hydroxyl group of the growing chain at the 3'-position of the added monomer, which is usually a phosphorus derivative such as phosphotriester, phosphoramidite. See, for example, Matteuci et al., Tet. Lett. 521:719 (1980); U.S. Patent No. 4,500,707 to Caruthers et al.; and U.S. Patent Nos. 5,436,327 and 5,700,637 to Southern et al. In another aspect, the present application provides a vector comprising the isolated mpolynucleotide according to the present application. The vector can be any linear nucleic acid, plasmid, phagemid, cosmid, RNA vector, viral vector, etc. Non-limiting examples of viral vectors may include retroviruses, adenoviruses, and adeno-associated viruses. In some embodiments, the vector is an expression vector, e.g., a phage display vector.

In another aspect, the present application provides one or more vectors comprising the nucleic acid molecules. For example, the vector may comprise one or more nucleic acid molecules according to the present application. One or more of the nucleic acid molecules may be comprised in each vector. In addition, other genes may be comprised in the vector, such as marker genes that allow selection of the vector in appropriate host cells and under appropriate conditions. In addition, the vector may comprise expression control elements that allow correct expression of the coding region in an appropriate host. Such control elements are well known to those skilled in the art, and may include, for example, promoters, ribosome binding sites, enhancers, and other control elements that regulate gene transcription or mRNA translation. In some embodiments, the expression control sequences are regulatable elements. The specific structure of the expression control sequence can vary depending on species or the function of cell types, but generally includes 5' non-transcribed sequences and 5' and 3' non-translated sequences involved in the initiation of transcription and translation, respectively, such as TATA box, caped sequence, CAAT sequence, respectively. For example, the 5' non-transcribed expression control sequence may comprise a promoter region, and the promoter region may comprise a promoter sequence for transcriptional control of the functionally linked nucleic acid. The expression control sequences may also include enhancer sequences or upstream activator sequences. In the present application, suitable promoters may include, for example, promoters for SP6, T3 and T7 polymerases, human U6 RNA promoters, CMV promoters and artificial hybrid promoters thereof (such as CMV), wherein a certain part of the promoter can be fused with a certain part of the gene promoter of other cellular proteins (such as human GAPDH, glyceraldehyde-3-phosphate dehydrogenase). It may or may not contain additional introns. One or more nucleic acid molecules according to the present application can be operably linked to the expression control element.

The vector may include, for example, a plasmid, a cosmid, a virus, a phage, or other vectors commonly used in, for example, genetic engineering. In some embodiments, the vector may be an expression vector.

The vector may further comprise one or more selective marker genes which, when expressed, confer one or more phenotypic traits that can be used to select or otherwise identify host cells carrying the vector. Non-limiting examples of suitable selective markers for eukaryotic cells include dihydrofolate reductase and neomycin resistance.

In another aspect, the present application provides a cell comprising the fusion protein, the immunoconjugate, the nucleic acid molecule or the vector. The cell may be a host cell. For example, the cell may include many cell types, for example, prokaryotic cells such as E. coli or Bacillus subtilis cells, fungal cells such as yeast cells or Aspergillus cells, insect cells such as S2 Drosophila cells or Sf9 cells, or animal cells such as fibroblasts, CHO cells, COS cells, NSO cells, HeLa cells, BHK cells, HEK 293 cells or human cells.

For example, the vector can be introduced into a host cell stably or transiently by a variety of established techniques. For example, one method involves calcium chloride treatment, in which the vector is introduced by calcium precipitation. Other salts, such as calcium phosphate, may be used in a similar manner. Alternatively, electroporation (i.e., application of electrical current to increase the permeability of cells to nucleic acids) can be used. Other examples of transformation methods include microinjection, DEAE dextran-mediated transformation, and heat shock in the presence of lithium acetate. Lipoplexes, liposomes, and dendrimers can also be used to transfect host cells.

When introducing heterologous sequences into host cells, a variety of methods can be implemented to identify host cells into which the vector has been introduced. An exemplary selection method involves subculturing single cells to form single colonies and then testing for expression of the desired protein product. Another approach requires the selection of host cells containing heterologous sequences based on the phenotypic traits conferred by expression of a selective marker gene contained within the vector.

For example, the introduction of the various heterologous sequences of the present application into a host cell can be confirmed by methods such as PCR, Southern blotting, or Northern blot hybridization. For example, nucleic acids can be prepared from the resulting host cells, and the specific target sequence can be amplified by PCR using primers specific for the target sequence. The amplification product is subjected to agarose gel electrophoresis, polyacrylamide gel electrophoresis or capillary electrophoresis, and then stained with ethidium bromide, SYBR Green solution, etc., or UV detection is used to detect DNA. Alternatively, nucleic acid probes specific for the target sequence can be used in the hybridization reaction. Expression of a specific gene sequence can be determined by PCR, Northern blot hybridization, or by detection of the corresponding mRNA by reverse transcription by immunoassays using antibodies that react with the encoded gene product. Exemplary immunoassays include, but are not limited to, ELISA, radioimmunoassay, and sandwich immunoassay.

In addition, the introduction of various heterologous sequences of the present application into a host cell can be confirmed by the enzymatic activity of an enzyme (e.g., an enzymatic marker) encoded by the heterologous sequence. Enzymes can be determined by a variety of methods known in the art. Typically, enzymatic activity can be determined by the formation of products or conversion of the substrate of the enzymatic reaction under investigation. The reaction can be carried out in vitro or in vivo.

In another aspect, the present application provides a method for preparing the fusion protein, which may include culturing the cells under conditions enabling the expression of the fusion protein. For example, appropriate culture medium, appropriate temperature and culture time can be used, and these methods are understood by those of ordinary skill in the art.

In some cases, the method may further include a step of isolating and/or purifying the fusion protein. For example, affinity chromatography can be performed using protein G-agarose or protein A-agarose, and the fusion protein according to the present application can also be purified and isolated by gel electrophoresis and/or high performance liquid chromatography.

### Compositions and applications

In another aspect, the present application provides a composition comprising the fusion protein, the immunoconjugate or the nucleic acid molecule, and optionally a pharmaceutically acceptable excipient.

For example, the pharmaceutically acceptable excipient may include buffers, antioxidants, preservatives, low molecular weight polypeptides, proteins, hydrophilic polymers, amino acids, sugars, chelating agents, counterions, metal complexes, and/or nonionic surfactants, etc.

In the present application, the composition may be formulated with a pharmaceutically acceptable carrier or diluent and any other known adjuvants and excipients according to conventional techniques in the art, for example, according to the technique disclosed in Remington: The Science and Practice of Pharmacy, Nineteenth Edition, edited by Gennaro, Mack Publishing Co., Easton, PA, 1995.

In the present application, the composition can be formulated for oral administration, intravenous administration, intramuscular administration, in situ administration at the tumor site, inhalation, rectal administration, vaginal administration, transdermal administration or administration via a subcutaneous depot.

For example, the composition can be used to inhibit tumor growth. For example, the composition of the present application can inhibit or delay the development or progression of a disease, can reduce the size of a tumor (or even substantially eliminate a tumor), and/or can alleviate and/or stabilize a disease state.

For example, the composition according to the present application may be in a form suitable for oral administration, such as tablets, capsules, pills, powders, sustained-release preparations, solutions, suspensions, or for parenteral injection, such as sterile solutions, suspensions or emulsions, or for topical administration as ointments or creams, or for rectal administration as suppositories. The compositions may be in unit dose form suitable for single administration of precise doses. The composition may further comprise conventional pharmaceutical carriers or excipients. In addition, the composition may include other drugs or agents, carriers, adjuvants, and the like.

The composition according to the present application may comprise a therapeutically effective amount of the fusion protein. The therapeutically effective amount is the dose required to prevent and/or treat (at least in part) the disease or disorder (e.g., tumor) and/or any complications thereof in a subject suffering from or at risk of developing it. The specific amount/concentration of the dose may vary depending on the method of administration and the patient's needs, and may be determined based on, for example, the patient's volume, viscosity and/or body weight, etc. It will be understood that these specific doses may be readily adjusted by one of skill in the art (e.g., a physician or pharmacist) based on the particular patient, formulation, and/or disease conditions.

In the present application, the terms "treatment" or "healing" or "relief" or "improvement" are used interchangeably in the present application and refer to methods of obtaining beneficial or desired results, including but not limited to therapeutic and/or preventive benefits. In the present application, a therapeutic benefit generally refers to eradication or lessening of the severity of the underlying disorder being treated. Also, a therapeutic benefit is achieved with the eradication, lessening of the severity, or reduction in the incidence of one or more physiological symptoms associated with the underlying disorder such that an improvement is observed in the subject (notwithstanding that the subject may still be afflicted with the underlying disorder). For prophylactic benefits, the composition may be administered to subjects who are at risk of developing a particular disease, or who report one or more physiological symptoms of a disease, even though a diagnosis of the disease may not have been made.

In another aspect, the present application provides the use of the fusion protein, the immunoconjugate, the nucleic acid molecule, the vector, the composition, or the cell in the preparation of drugs for the prevention and/or treatment of diseases and/or disorders.

In another aspect, the present application provides the fusion proteins, immunoconjugates, nucleic acid molecules, vectors, compositions or cells for preventing and/or treating diseases and/or disorders.

In another aspect, the present application provides a method for preventing and/or treating diseases and/or disorders, comprising administering to a subject the fusion protein, immunoconjugate, nucleic acid molecule, vector, composition or cell according to the present application.

In another aspect, the present application provides a method of inhibiting the growth and/or proliferation of tumors or tumor cells, which may include bringing the fusion protein or composition according to the present application into contact with the tumor or tumor cells. For example, the contact may occur in vitro.

In another aspect, the present application provides a method that can inhibit the growth and/or proliferation of tumors or tumor cells, which can include administering an effective amount of the fusion protein, the immunoconjugate, the nucleic acid molecule, the vector, the composition, or the cell to a subject in need thereof.

In the present application, the diseases and/or disorders may include diseases and/or disorders associated with CLDN18.2.

In the present application, the diseases and/or disorders associated with CLDN18.2 include diseases involving cells expressing CLDN18.2 or diseases associated with cells expressing CLDN18.2.

In the present application, the tumor may include solid tumors and non-solid tumors.

In the present application, the tumor may include gastric cancer, esophageal cancer, pancreatic cancer, lung cancer, ovarian cancer, colon cancer, liver cancer, head and neck cancer, and/or gallbladder cancer.

In another aspect, the present application provides the fusion protein, the immunoconjugate, the nucleic acid molecule, the vector, the composition, or the cell for preventing and/or treating diseases and/or disorders.

In the present application, the term "subject" generally refers to human or non-human animals, including but not limited to cats, dogs, horses, pigs, cattle, sheep, goats, rabbits, mice, rats or monkeys.

Without wishing to be bound by any theory, the following examples are only for illustrating the various technical solutions of the present application, and are not used to limit the scope of the invention of the present application.

### EXAMPLES

### Example 1 Construction of fusion protein

Referring to the fusion protein structure shown in Fig. 1, taking CS001 as an example, from N-terminus to C-terminus, using linker 1 (with the amino acid sequence as set forth in SEQ ID NO: 29), Claudin18.2 nanoantibody NA3S-H1, the linker and SIRPα mutant M91N (with the amino acid sequence as set forth in SEQ ID NO: 12) were sequentially linked, wherein the N-terminus of M91N and the C-terminus of the heavy chain of NA3S-H1 were linked through the linker to obtain the fusion protein CS001.

CS002, CS003, CS004, CS005, and CS006 are based on CS001, except that M91N is replaced with the corresponding SIRPα mutants 002N, M5N, M169N, M82N, and M84N according to Table 1. Among them, 002N is obtained by performing N110A mutation on the amino acid sequences set forth in SEQ ID NO: 23.

The amino acid sequences of HCDR1-3 of nanobody NA3S-H1 are as set forth in SEQ ID NO: 27, SEQ ID NO: 26 and SEQ ID NO: 25 respectively, and the amino acid sequence of VHH is as set forth in SEQ ID NO: 24, respectively. The amino acid sequence of IgG Fc is as set forth in SEQ ID NO: 28.

**Table 1 Composition of the fusion proteins**

| No. | First binding domain | Second binding domain | Amino acid sequence |
|---|---|---|---|
| CS001 | NA3S-H1 | M91N | SEQ ID NO: 30 |
| CS002 | NA3S-H1 | 002N | SEQ ID NO: 31 |
| CS003 | NA3S-H1 | M5N | SEQ ID NO: 32 |
| CS004 | NA3S-H1 | M169N | SEQ ID NO: 33 |
| CS005 | NA3S-H1 | M82N | SEQ ID NO: 34 |
| CS006 | NA3S-H1 | M84N | SEQ ID NO: 35 |

### Example 2 Detection of binding activity to dual antigens

### (1) Evaluation of the binding activity of bifunctional fusion protein to CD47 by ELISA.

The ELISA strip plate was coated with CD47 (CD47 Protein, Human, Recombinant (ECD, His Tag), Sino Biological) and left overnight at 4°C; after washing with PBST, 10% fetal bovine serum was added to block at 37°C for 1 h; different concentrations of bifunctional fusion proteins CS001, CS002, CS003, CS004, CS005 and CS006 were added, and the system was allowed to react at 37°C for 1 h; after washing with PBST, horseradish peroxidase-labeled goat anti-human IgG Fc secondary antibody (Goat anti-human IgG Fc antibody, horseradish peroxidase (HRP) conjugate, affinity purified, Invitrogen) was added, the system was allowed to react at 37°C for 30 min; it was washed 5 times with PBST; 100 µL of TMB (eBioscience) was added to each well, and the plate was placed at room temperature (20±5°C) in the dark for 1-2 min; then 100 µL of 2N H₂SO4 stop buffer was added to each cell to stop the substrate reaction, the OD value was read at 450 nm with a microplate reader, and the binding ability of the bifunctional fusion protein to CD47 was analyzed.

The results are shown in Fig. 2. Fig.e 2 shows that the bifunctional fusion protein CS001 has the strongest binding ability to CD47, while CS002 and CS003 have similar and weakest binding ability to CD47.

### (2) Evaluation of the binding activity of the bifunctional fusion protein to CD47-positive cell line Raji cells by flow cytometry.

Human lymphoma tumor cells Raji were collected, and added into a 1.5 mL EP tube at 5×10⁵ cells per tube; different concentrations of bifunctional fusion proteins CS001, CS002, CS003, CS004, CS005 and CS006 were added, and the system was incubated on ice in the dark for 30 min; after washing with FACS wash buffer, PE fluorescently labeled Goat Anti-Human IgG Fc Secondary Antibody (Invitrogen) was added and the system was incubated on ice in the dark for 30 min; the system was washed with FACS wash buffer 2 times; to each tube, 400 µL of 1% paraformaldehyde fixative (Solarbio) was added to fix the cells. After mixing, the system was detected for the relative fluorescence intensity of PE fluorescence and the positive rate on the machine, and the binding ability of the bifunctional fusion proteins CS001, CS002, CS003, CS004, CS005 and CS006 to Raji cells was analyzed.

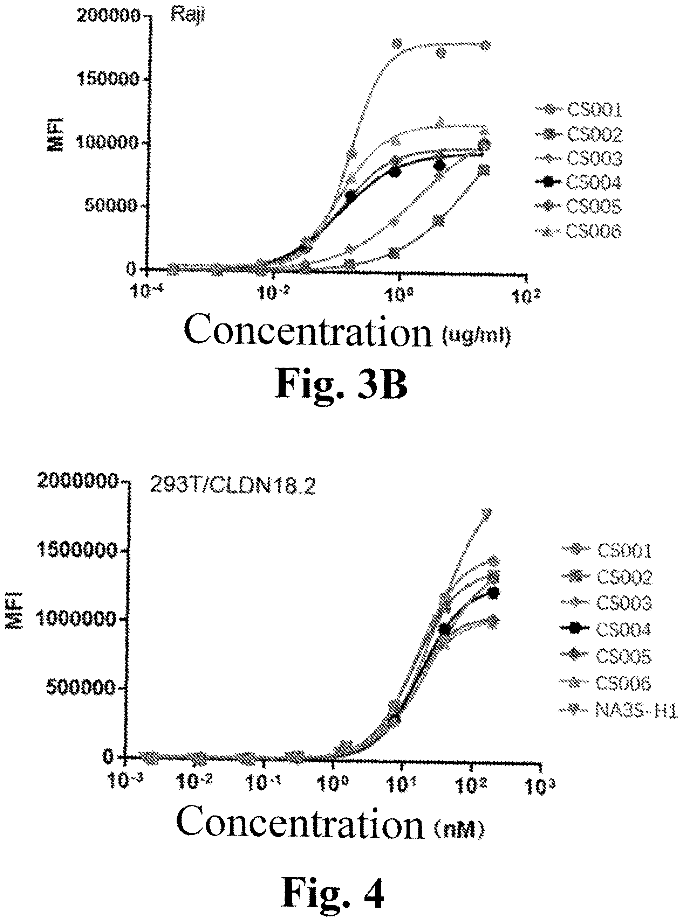

The results are shown in Figs. 3A-3B. Figs. 3A-3B show that the bifunctional fusion protein CS001 has the strongest binding ability to CD47-positive tumor cells Raji, while CS002 has the weakest binding ability to Raji cells.

### (3) Using the Claudin18.2 nanoantibody NA3S -H1as a control, evaluation of the binding activity of bifunctional fusion proteins to HEK-293T cells overexpressing the human Claudin18.2 gene (abbreviated as 293T/CLDN18.2) by flow cytometry.

293T/CLDN18.2 cells were collected, and added into a 1.5 mL EP tube at 5×10⁵ cells per tube; different concentrations of bifunctional fusion proteins CS001, CS002, CS003, CS004, CS005 and CS006 and Claudin18.2 nanoantibody NA3S-H1 were added, and the system was incubated on ice in the dark for 30 min; after washing with FACS wash buffer, PE fluorescently labeled Goat Anti-Human IgG Fc Secondary Antibody (Invitrogen) was added and the system was incubated on ice in the dark for 30 min; the system was washed with FACS wash buffer 2 times; to each tube, 400µL of 1% paraformaldehyde fixative (Solarbio) was added to fix the cells. After mixing, the system was detected for the relative fluorescence intensity of PE fluorescence and the positive rate on the machine, and the binding ability of the bifunctional fusion proteins CS001, CS002, CS003, CS004, CS005 and CS006 to 293T/CLDN18.2 cells was analyzed.

The results are shown in Fig. 4. Fig. 4 shows that all bifunctional fusion proteins have similar binding ability to 293T/CLDN18.2 cells, as does the nanoantibody NA3S-H1.

### Example 3 Analysis of the activity of blocking CD47/SIRPα interaction

The biological activity of the bifunctional fusion protein in blocking CD47/SIRPα interaction was evaluated by using ELISA.

The ELISA plate was coated with SIRPα (Recombinant Human SIRPA, Sino Biological), 1ug/ml, left overnight at 4°C; after washing with PBST, 10% fetal bovine serum was added to block at 37°C for 1 h; different concentrations of bifunctional fusion proteins CS001, CS002, CS003, CS004, CS005 and CS006 were added to react at 37°C for 1 h; after washing with PBST, biotin marker Biotin-CD47 (Recombinant Human CD47-biotin, Jiaxuan Biotech) was added to a final concentration of 2 ug/ml, the system was allowed to react at 37°C for 30 min; it was washed 5 times with PBST; horseradish peroxidase-labeled avidin (Streptavidin-HRP, Jiaxuan Biotech) was added, the system was incubated at 37°C for 30 min, and washed 5 times with PBST; 100 µL of TMB (eBioscience) was added to each well, the plate was placed at room temperature (20±5°C) in the dark for 1-5 min; 100 µL 2N H₂SO4 stop buffer was added to each well to stop the substrate reaction, the OD value was read at 450 nm with a microplate reader, and the blocking effects of the bifunctional fusion proteins CS001, CS002, CS003, CS004, CS005 and CS006 on CD47/SIRPα were analyzed.

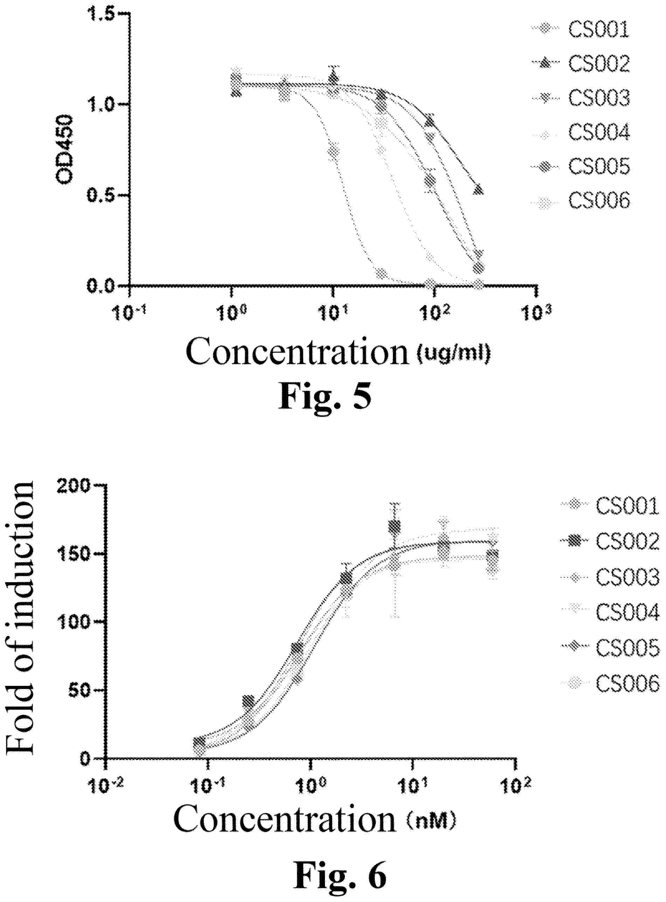

As can be seen from Fig. 5, the bifunctional fusion protein CS001 has the strongest ability to competitively block the binding of CD47 and S1RPα, while CS002 has the weakest blocking ability.

### Example 4 ADCC activity analysis

Using Claudin18.2 nanoantibody NA3S-H1 as control, Claudin18.2 positive cell line 293T/CLDN18.2 as target cells, and Jurkat cells (Jurkat-ADCC cells for short) overexpressing human FcyRIIIa gene and NFAT fluorescent reporter gene as effector cells, the ADCC activity of the bifunctional fusion protein was evaluated by using the reporter gene method.

293T/CLDN18.2 cells were collected and added to a 96-well cell culture plate at 2×10⁴ cells per tube; Jurkat-ADCC cells were collected and added to a 96-well cell culture plate at 1.2×10⁵ per tube; different concentrations of functional fusion proteins CS001, CS002, CS003, CS004, CS005 and CS006, and Claudin18.2 nanoantibody NA3S-H1 were added, and the system was incubated in a 37°C incubator for 6 h; 100 µL of luciferase detection solution was added to each well, the system was allowed to react at room temperature in the dark for 10 min, then the relative fluorescence intensity (RLU) of chemiluminescence was read using a microplate reader, and the ADCC activity of the bifunctional fusion proteins CS001, CS002, CS003, CS004, CS005 and CS006 was analyzed.

The results are shown in Fig. 6. Fig. 6 shows that all bifunctional fusion proteins have similar ADCC activity, and their activity is the same as that of nanoantibody NA3S-H1.

### Example 5 Analysis of binding activity to red blood cells

The binding activity of bifunctional fusion proteins to healthy human red blood cells was evaluated by flow cytometry.

Healthy human red blood cells were collected, and added into a 1.5 mL EP tube at 1×10⁶ cells per tube; different concentrations of bifunctional fusion proteins CS001, CS002, CS003, CS004, CS005 and CS006 were added, and the system was incubated on ice in the dark for 30 min; after washing with FACS wash buffer, PE fluorescently labeled Goat Anti-Human IgG Fc Secondary Antibody (Invitrogen) was added and the system was incubated on ice in the dark for 30 min; the system was washed with FACS wash buffer 2 times; to each tube, 400 µL of 1% paraformaldehyde fixative (Solarbio) was added to fix the cells. After mixing, the system was detected for the relative fluorescence intensity of PE fluorescence and the positive rate on the machine, and the binding ability of the bifunctional fusion proteins CS001, CS002, CS003, CS004, CS005 and CS006 to human red blood cells was analyzed.

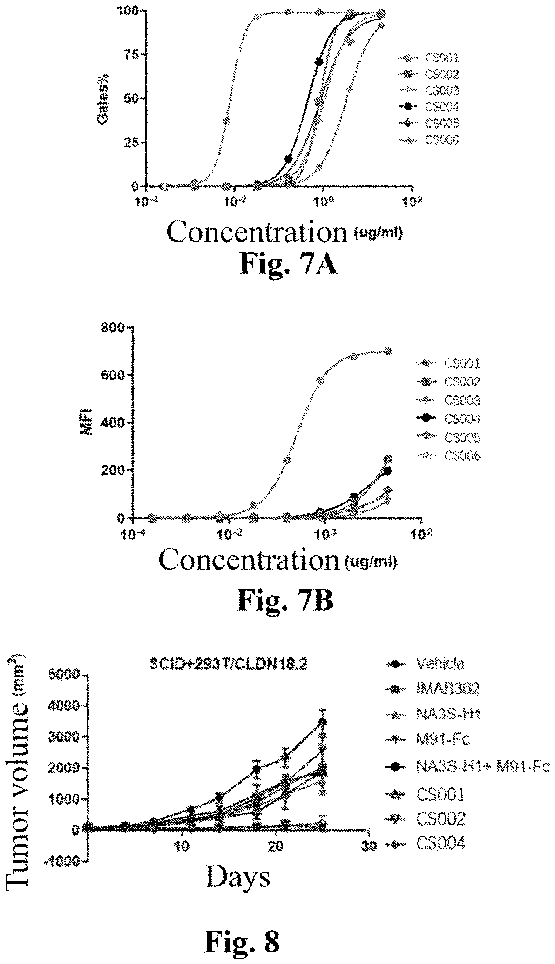

The results are shown in Figs. 7A-7B. Figs. 7A-7B show that all the bifunctional fusion proteins can bind to red blood cells to varying degrees. CS001 has the strongest ability to bind to red blood cells, and CS003 has the weakest ability to bind to red blood cells.

### Example 6 Bifunctional fusion protein inhibits tumor activity in vivo

The effect of the bifunctional fusion protein in inhibiting tumor activity was evaluated by using the allograft SCID mouse model of HEK-293T cells overexpressing the Claudin18.2 gene (293T/CLDN18.2).

293T/CLDN18.2 cells were inoculated subcutaneously into the right shoulder of female SCID mice. A total of 24 mice were inoculated. When the tumor grew to about 100 mm³, the mice were grouped and administered with drugs. There were 8 groups in total, with 3 mice in each group, including the Vehicle group, IMAB362 (20 mg/kg) group, NA3S-H1 (10 mg/kg) group, M91-Fc (10 mg/kg) group, NA3S-H1 + M91-Fc (10+10 mg/kg) group, CS001 (15 mg/kg) group, CS002 (15 mg/kg) group, and CS004 (15 mg/kg) group. The drugs were administered by tail vein injection twice a week for three weeks. The tumor volume and body weight were measured every week, and the relationship between the changes in body weight and tumor volume of the tumor-bearing mice and the administration time was recorded. At the end of the experiment, the tumor-bearing mice were euhanized, and the tumors were peeled off, weighed, and photographed. The tumor growth inhibition rate TGITV (%) was calculated and statistically analyzed.

The results are shown in Fig. 8. Fig. 8 shows that the tumor growth inhibition rates of the IMAB362, NA3S-H1,M91-Fc, NA3S-H1 +M91-Fc, CS001, CS002, and CS004 groups are 40%, 54%, 36%, 53%, 34%, 96%, 97%, respectively. The tumor volume of each treatment group is significantly lower than that of the blank control group (p<0.05), showing a significant anti-tumor effect and effective inhibition of tumor growth. The tumor volumes of the CS002 and CS004 groups are similar and the smallest among all treatment groups, significantly lower than those of M91-Fc (p<0.01), NA3S-H1 (p<0.01) and NA3S-H1 + M91-Fc groups (p<0.05), showing that their tumor inhibitory activity is better than that of the single drug group and the combination group. During the treatment, the tumor-bearing mice showed good tolerance to the test substances IMAB362, NA3S-H1, M91-Fc, NA3S-H1 + M91-Fc, CS001, CS002, and CS004. The mice in each group had normal weight and no abnormal performance, generally in good condition.

The foregoing detailed description is provided by way of explanation and example, and is not intended to limit the scope of the appended claims. Various modifications to the embodiments according to the present application will be apparent to those of ordinary skill in the art and remain within the scope of the appended claims and their equivalents.

## Claims

1. A fusion protein, comprising:
a first binding domain that specifically binds to CLDN18.2; and
a second binding domain that specifically binds to CD47 protein;
wherein, the second binding domain has at least one of the following properties:
(a) binding to CD47 protein with a KD value of 4×10⁻⁸M or lower;
(b) specifically blocking the interaction between CD47 protein and SIRPα;
(c) not inducing coagulation; and
(d) inhibiting the growth and/or proliferation of tumors or tumor cells.

2. The fusion protein according to claim 1, wherein the second binding domain comprises a mutant of human SIRPα variant 1, and the mutant comprises an amino acid mutation at one or more positions compared with the 33rd-149th amino acid sequence of SEQ ID NO: 22.

3. The fusion protein according to claim 2, wherein the mutant comprises an amino acid mutation at one or more amino acid positions selected from the group consisting of: L44, 161, V63, E77, Q82, K83, E84, V93, D95, L96, K98, N100, R107, G109 and V132.

4. The fusion protein according to any one of claims 2-3, wherein the mutant comprises an amino acid substitution at I61 and E77 amino acid residues.

5. The fusion protein according to any one of claims 2-4, wherein the mutant comprises an amino acid substitution at I61 and V132 amino acid residues.

6. The fusion protein according to any one of claims 2-5, wherein the mutant comprises an amino acid substitution at I61 and E84 amino acid residues.

7. The fusion protein according to any one of claims 2-6, wherein the mutant comprises an amino acid substitution at I61, K83 and E84 amino acid residues.

8. The fusion protein according to any one of claims 2-7, wherein the mutant comprises an amino acid substitution at I61, G109 and V132 amino acid residues.

9. The fusion protein according to any one of claims 2-8, wherein the mutant comprises an amino acid substitution at I61, D95, L96, G109 and V132 amino acid residues.

10. The fusion protein according to any one of claims 2-9, wherein the mutant comprises an amino acid substitution at V93 and R107 amino acid residues.

11. The fusion protein according to any one of claims 2-10, wherein the mutant comprises an amino acid substitution at L44 and I61 amino acid residues.

12. The fusion protein according to any one of claims 2-11, wherein the mutant comprises an amino acid substitution at L44 and E77 amino acid residues.

13. The fusion protein according to any one of claims 2-12, wherein the mutant comprises an amino acid substitution at Q82, K83 and E84 amino acid residues.

14. The fusion protein according to any one of claims 2-13, wherein the mutant comprises an amino acid substitution at E77 and V132 amino acid residues.

15. The fusion protein according to any one of claims 2-14, wherein the mutant comprises an amino acid substitution at amino acid residues selected from the group consisting of:
(1) I61, V63, E77, E84, V93, L96, K98, N100 and V132;
(2) 161, E77, Q82, K83 and E84;
(3) 161, V63, K83, E84 and V132;
(4) 161, E77, E84, R107 and V132;
(5) 161, V63, E77, K83, E84 and N100;
(6) I61, E77, Q82, K83, E84 and R107;
(7) I61, E77, Q82, E84, V93, L96, N100, R107, G109 and V132;
(8) I61, E77, Q82, K83, E84 and V132;
(9) 161;
(10) I61, D95, L96, G109 and V132;
(11) 161, D95, L96, K98, G109 and V132;
(12) I61, E77, E84, V93, R107 and V132;
(13) E77, L96, N100, G109 and V132;
(14) I61, V63, Q82, E84, D95, L96, N100 and V132;
(15) 161, E77, Q82, K83, E84, V93, D95, L96, K98, N100 and V132;
(16) I61, E77, Q82, K83, E84 and V93;
(17) 161, V63, E77, K83, E84, D95, L96, K98 and N100;
(18) I61, V63, E77, K83, D95, L96, K98, N100 and G109;
(19) 161, E77, Q82, E84, V93, D95, L96, K98 and N100;
(20) 161, V63, E77, Q82 and E84; and
(21) L44, 161, E77, Q82, K83, E84 and V132.

16. The fusion protein according to any one of claims 2-15, wherein the mutant comprises one or more amino acid substitutions selected from the group consisting of: L44V, I61L/V/F, V63I, E77I/N/ Q/K/H/M/R/V/L, Q82S/R/G/N, K83R, E84Q/K/H/D/R/G, V93L/A, D95H/R/E, L96S/T, K98R, N100G/K/D/E, R107N/S, G109R/H and V132L/R/I/S.

17. The fusion protein according to any one of claims 2-16, wherein the mutant comprises amino acid substitutions of I61L/V/F and E77Q/N/I.

18. The fusion protein according to any one of claims 2-17, wherein the mutant comprises amino acid substitutions of I61L/V/F and V132VS.

19. The fusion protein according to any one of claims 2-18, wherein the mutant comprises amino acid substitutions of I61L/V/F, K83R and E84Q/D/H.

20. The fusion protein according to any one of claims 2-19, wherein the mutant comprises amino acid substitutions of I61L/V/F, G109H, and V132I/S.

21. The fusion protein according to any one of claims 2-20, wherein the mutant comprises amino acid substitutions of I61L/V/F, D95H, L96S, G109H and V132I/S.

22. The fusion protein according to any one of claims 2-21, wherein the mutant comprises amino acid substitutions of V93A and R107N.

23. The fusion protein according to any one of claims 2-22, wherein the mutant comprises amino acid substitutions of L44V and I61L/V/F.

24. The fusion protein according to any one of claims 2-23, wherein the mutant comprises amino acid substitutions of L44V and E77Q/N/I.

25. The fusion protein according to any one of claims 2-24, wherein the mutant comprises amino acid substitutions of Q82S/R/G/N, K83R and E84Q/K/H/D/R/G.

26. The fusion protein according to any one of claims 2-25, wherein the mutant comprises amino acid substitutions of E77Q/N/I and V132I/S.

27. The fusion protein according to any one of claims 2-26, wherein the mutant comprises amino acid substitutions selected from the group consisting of:
(1) I61L, V63I, E77I, E84K, V93L, L96S, K98R, N100G and V132L;
(2) I61V, E77N, Q82S, K83R and E84H;
(3) I61F, V63I, K83R, E84K and V132I;
(4) I61L, E77Q, E84D, R107N and V132I;
(5) I61L, V63I, E77K, K83R, E84D and N100G;
(6) I61V, E77H, Q82R, K83R, E84H and R107S;
(7) I61L, E77I, Q82G, E84R, V93L, L96T, N100G, R107S, G109R and V132R;
(8) I61L, E77M, Q82G, K83R, E84D and V132L;
(9) I61L;
(10) I61F, D95H, L96S, G109H and V132S;
(11) I61F, D95H, L96S, K98R, G109H and V132S;
(12) I61L, E77Q, E84D, V93A, R107N and V132I;
(13) E77K, L96S, N100K, G109H and V132L;
(14) I61L, V63I, Q82G, E84G, D95R, L96S, N100D and V1321;
(15) I61L, E77R, Q82N, K83R, E84G, V93L, D95E, L96T, K98R, N100D and V132L;
(16) I61V, E77N, Q82S, K83R, E84H and V93A;
(17) I61V, V63I, E77V, K83R, E84D, D95E, L96T, K98R and N100E;
(18) I61L, V63I, E77V, K83R, D95E, L96S, K98R, N100D and G109R;
(19) I61V, E77L, Q82G, E84G, V93L, D95E, L96T, K98R and N100G;
(20) I61L, V63I, E77N, Q82G and E84G, and
(21) L44V, I61F, E77I, Q82R, K83R, E84Q and V132I.

28. The fusion protein according to any one of claims 2-27, wherein the mutant further comprises one or more glycosylation site modifications.

29. The fusion protein according to claim 28, wherein the glycosylation site modification comprises a glycosylation site mutation.

30. The fusion protein according to claim 29, wherein the glycosylation site mutation is located at the N110 position.

31. The fusion protein according to any one of claims 29-30, wherein the glycosylation site mutation comprises the amino acid substitution of N110A.

32. The fusion protein according to any one of claims 2-31, wherein the mutant comprises the amino acid sequences set forth in any one of SEQ ID NOs: 1-21.

33. The fusion protein according to any one of claims 1-33, wherein the first binding domain comprises an antigen-binding protein targeting CLDN18.2.

34. The fusion protein according to claim 33, wherein the antigen-binding protein comprises an antibody or an antigen-binding fragment thereof.

35. The fusion protein according to claim 34, wherein the antibody is selected from the group consisting of a monoclonal antibody, a single-chain antibody, a chimeric antibody, a humanized antibody and a fully human antibody.

36. The fusion protein according to any one of claims 34-35, wherein the antigen-binding fragment is selected from the group consisting of: Fab, Fab', F(ab)2, dAb, isolated complementarity determining region CDR, Fv and scFv.

37. The fusion protein according to any one of claims 1-36, wherein the CLDN18.2 comprises human CLDN18.2.

38. The fusion protein according to any one of claims 1-37, wherein the first binding domain comprises at least one CDR in a heavy chain variable region (VH), and the VH comprises the amino acid sequences set forth in SEQ ID NO: 24.

39. The fusion protein according to any one of claims 1-38, wherein the first binding domain comprises HCDR3, and the HCDR3 comprises the amino acid sequences set forth in SEQ ID NO: 25.

40. The fusion protein according to any one of claims 1-39, wherein the first binding domain comprises HCDR2, and the HCDR2 comprises the amino acid sequences set forth in SEQ ID NO: 26.

41. The fusion protein according to any one of claims 1-40, wherein the first binding domain comprises HCDR1, and the HCDR1 comprises the amino acid sequences set forth in SEQ ID NO: 27.

42. The fusion protein according to any one of claims 1-41, wherein the first binding domain comprises VH, and the VH comprises the amino acid sequences set forth in SEQ ID NO: 24.

43. The fusion protein according to any one of claims 1-42, wherein the first binding domain comprises an immunoglobulin single variable domain.

44. The fusion protein according to claim 43, wherein the immunoglobulin single variable domain comprises VHH.

45. The fusion protein according to any one of claims 1-44, wherein the first binding domain comprises at least one CDR in VHH, and the VHH comprises the amino acid sequences set forth in SEQ ID NO: 24.

46. The fusion protein according to any one of claims 44-45, wherein the VHH comprises HCDR3, and the HCDR3 comprises the amino acid sequences set forth in SEQ ID NO: 25.

47. The fusion protein according to any one of claims 44-46, wherein the VHH comprises HCDR2, and the HCDR2 comprises the amino acid sequences set forth in SEQ ID NO: 26.

48. The fusion protein according to any one of claims 44-47, wherein the VHH comprises HCDR1, and the HCDR1 comprises the amino acid sequences set forth in SEQ ID NO: 27.

49. The fusion protein according to any one of claims 44-48, wherein the VHH comprises the amino acid sequences set forth in SEQ ID NO: 24.

50. The fusion protein according to any one of claims 1-49, wherein the first binding domain comprises a heavy chain constant region Fc fragment.

51. The fusion protein according to claim 50, wherein the heavy chain constant region is derived from IgG.

52. The fusion protein according to any one of claims 50-51, wherein the heavy chain constant region is derived from human IgG.

53. The fusion protein according to any one of claims 51-52, wherein the IgG is selected from the group consisting of IgG1 and IgG4.

54. The fusion protein according to any one of claims 50-53, wherein the heavy chain constant region Fc segment comprises the amino acid sequences set forth in SEQ ID NO: 28.

55. The fusion protein according to any one of claims 1-54, wherein the first binding domain is located at the N-terminus of the second binding domain.

56. The fusion protein according to any one of claims 1-55, wherein the fusion protein further comprises a linker located at the C-terminus of the first binding domain and at the N-terminus of the second binding domain.

57. The fusion protein according to claim 56, wherein the linker comprises the amino acid sequences set forth in SEQ ID NO: 29.

58. The fusion protein according to any one of claims 1-57, comprising at least 2 second binding domains.

59. The fusion protein according to claim 58, wherein each of the second binding domains is located at the C-terminus of the first binding domain, respectively.

60. The fusion protein according to any one of claims 1-59, comprising at least one polypeptide chain, wherein the polypeptide chain comprises the amino acid sequences set forth in any one of SEQ ID NOs: 30-35.

61. The fusion protein according to any one of claims 1-60, comprising two polypeptide chains comprising the same amino acid sequence.

62. An immunoconjugate comprising the fusion protein according to any one of claims 1-61.

63. One or more isolated nucleic acid molecules encoding the fusion protein according to any one of claims 1-61 or the immunoconjugate according to claim 62.

64. A vector, comprising the nucleic acid molecule according to claim 63.

65. A pharmaceutical composition, comprising the fusion protein according to any one of claims 1-61, the immunoconjugate according to claim 62, the nucleic acid molecule according to claim 63, and optionally a pharmaceutically acceptable carrier.

66. A cell, comprising the fusion protein according to any one of claims 1-61, the immunoconjugate according to claim 62, the nucleic acid molecule according to claim 63, or the vector according to claim 64.

67. A method for preparing the fusion protein according to any one of claims 1-61, comprising culturing the cell according to claim 66 under conditions that allow the expression of the fusion protein.

68. Use of the fusion protein according to any one of claims 1-61, the immunoconjugate according to claim 62, the nucleic acid molecule according to claim 63, the vector according to claim 64, the pharmaceutical composition according to claim 65, or the cell according to claim 66 in the preparation of a medicament for preventing and/or treating diseases and/or disorders.

69. The use according to claim 68, wherein the disease and/or disorder comprises a disease and/or disorder associated with CLDN18.2.

70. The use according to any one of claims 68-69, wherein the disease and/or disorder associated with CLDN18.2 includes diseases involving cells expressing CLDN18.2 or diseases associated with cells expressing CLDN18.2.

71. The use according to any one of claims 68-70, wherein the disease and/or disorder includes tumors.

72. The use according to claim 71, wherein the tumor includes solid tumors and/or non-solid tumors.

73. The use according to any one of claims 71-72, wherein the tumor includes gastric cancer, esophageal cancer, pancreatic cancer, lung cancer, ovarian cancer, colon cancer, liver cancer, head and neck cancer and/or gallbladder cancer.
